# EUROPEAN PATENT APPLICATION

(11) **EP 2 796 137 A1**
(43) Date of publication of application: **29.10.2014**
(21) Application number: 13164706.7
(22) Date of filing: 22.04.2013
(51) Int. Cl.: A61K 31/343, A61K 45/06, A61K 31/365, A61K 31/5415, A61P 35/00

(54) **SERCA inhibitor and Calmodulin antagonist combination**

(71) Applicant: Universität des Saarlandes, 66123 Saarbrücken (DE)
(72) Inventor: Zimmermann, Richard, 66424 Homburg (DE); Greiner, Markus, 66773 Schwalbach (DE); Linxweiler, Johannes, 66606 St. Wendel (DE); Linxweiler, Maximilian, 66606 St. Wendel (DE)
(74) Representative: Schiweck, Weinzierl & Koch

(57) **Abstract**

The present invention relates to novel combinations of active ingredients for use in the prevention and/or treatment of tumors. The tumors treated by the composition according to the invention overexpress *SEC62* gene and overproduce Sec62 protein. The combination of active ingredients comprises at least a SERCA inhibitor and at least a Calmodulin antagonist.

SEQUENCE LISTING

<110>  Universitaet des Saarlandes

<120>  SERCA inhibitor and Calmodulin antagonist combination

<130>  SAR14655EP

<140>  EP13164706.7
<141>  2013-4-22

<160>  20

<170>  PatentIn version 3.5

<210>  1
<211>  21
<212>  DNA
<213>  Unknown

<220>
<223>  Sec61-UTR siRNA


<220>
<221>  scRNA
<222>  (1)..(21)
<223>  siRNA

<400>  1
cguaaagugu auucuguact t                                                21


<210>  2
<211>  21
<212>  DNA
<213>  Unknown

<220>
<223>  SEC 62 siRNA


<220>
<221>  scRNA
<222>  (1)..(21)
<223>  siRNA

<400>  2
ggcuguggcc aaguaucuut t                                                21


<210>  3
<211>  21
<212>  DNA
<213>  Unknown

<220>
<223>  SEC61A1 siRNA


<220>
<221>  scRNA
<222>  (1)..(21)

<223>  siRNA

<400>  3
ggaauuugcc ugcuaaucat t                                                21


<210>  4
<211>  12
<212>  PRT
<213>  Unknown

<220>
<223>  N-terminus of Sec61


<220>
<221>  MISC_FEATURE
<222>  (1)..(12)
<223>  synthesized peptide

<400>  4

Ala Ile Lys Phe Leu Glu Val Ile Lys Pro Phe Cys
1               5                   10


<210>  5
<211>  14
<212>  PRT
<213>  Unknown

<220>
<223>  N-terminus of TRAM


<220>
<221>  MISC_FEATURE
<222>  (1)..(14)
<223>  synthesized peptide

<400>  5

Val Leu Ser His Glu Phe Glu Leu Gln Asn Gly Ala Asp Cys
1               5                   10


<210>  6
<211>  12
<212>  PRT
<213>  Unknown

<220>
<223>  Sec61a peptide


<220>
<221>  MISC_FEATURE
<222>  (1)..(12)
<223>  synthesized peptide

<400>  6

Met Ala Ile Lys Phe Leu Glu Val Ile Lys Pro Phe

1                   5                       10


<210>  7
<211>  12
<212>  PRT
<213>  Unknown

<220>
<223>  Sec61a peptide


<220>
<221>  MISC_FEATURE
<222>  (1)..(12)
<223>  synthesized peptide

<400>  7

Ile Lys Phe Leu Glu Val Ile Lys Pro Phe Cys Val
1                   5                       10


<210>  8
<211>  12
<212>  PRT
<213>  Unknown

<220>
<223>  Sec61a peptide


<220>
<221>  MISC_FEATURE
<222>  (1)..(12)
<223>  synthesized peptide

<400>  8

Phe Leu Glu Val Ile Lys Pro Phe Cys Val Ile Leu
1                   5                       10


<210>  9
<211>  12
<212>  PRT
<213>  Unknown

<220>
<223>  Sec61a peptide


<220>
<221>  MISC_FEATURE
<222>  (1)..(12)
<223>  synthesized peptide

<400>  9

Glu Val Ile Lys Pro Phe Cys Val Ile Leu Pro Glu
1                   5                       10

<210>  10
<211>  12
<212>  PRT
<213>  Unknown

<220>
<223>  Sec61a peptide


<220>
<221>  MISC_FEATURE
<222>  (1)..(12)
<223>  synthesized peptide

<400>  10

Ile Lys Pro Phe Cys Val Ile Leu Pro Glu Ile Gln
1               5                   10


<210>  11
<211>  12
<212>  PRT
<213>  Unknown

<220>
<223>  Sec61a peptide


<220>
<221>  MISC_FEATURE
<222>  (1)..(12)
<223>  synthesized peptide

<400>  11

Pro Phe Cys Val Ile Leu Pro Glu Ile Gln Lys Pro
1               5                   10


<210>  12
<211>  12
<212>  PRT
<213>  Unknown

<220>
<223>  Sec61a peptide


<220>
<221>  MISC_FEATURE
<222>  (1)..(12)
<223>  synthesized peptide

<400>  12

Cys Val Ile Leu Pro Glu Ile Gln Lys Pro Glu Arg
1               5                   10


<210>  13
<211>  12
<212>  PRT

<213>  Unknown

<220>
<223>  Sec61a peptide


<220>
<221>  MISC_FEATURE
<222>  (1)..(12)
<223>  synthesized peptide

<400>  13

Ile Leu Pro Glu Ile Gln Lys Pro Glu Arg Lys Ile
1               5                   10


<210>  14
<211>  12
<212>  PRT
<213>  Unknown

<220>
<223>  Sec 61a peptide


<220>
<221>  MISC_FEATURE
<222>  (1)..(12)
<223>  synthesized peptide

<400>  14

Pro Glu Ile Gln Lys Pro Glu Arg Lys Ile Gln Phe
1               5                   10


<210>  15
<211>  12
<212>  PRT
<213>  Unknown

<220>
<223>  Sec61a peptide


<220>
<221>  MISC_FEATURE
<222>  (1)..(12)
<223>  synthesized peptide

<400>  15

Ile Gln Lys Pro Glu Arg Lys Ile Gln Phe Lys Glu
1               5                   10


<210>  16
<211>  12
<212>  PRT
<213>  Unknown

<220>

<223>  Sec61a peptide

<220>
<221>  MISC_FEATURE
<222>  (1)..(12)
<223>  synthesized peptide

<400>  16

Lys Pro Glu Arg Lys Ile Gln Phe Lys Glu Lys Val
1               5                   10

<210>  17
<211>  12
<212>  PRT
<213>  Unknown

<220>
<223>  Sec61a peptide

<220>
<221>  MISC_FEATURE
<222>  (1)..(12)
<223>  synthesized peptide

<400>  17

Glu Arg Lys Ile Gln Phe Lys Glu Lys Val Leu Trp
1               5                   10

<210>  18
<211>  12
<212>  PRT
<213>  Unknown

<220>
<223>  Sec61a peptide

<220>
<221>  MISC_FEATURE
<222>  (1)..(12)
<223>  synthesized peptide

<400>  18

Lys Ile Gln Phe Lys Glu Lys Val Leu Trp Thr Ala
1               5                   10

<210>  19
<211>  399
<212>  PRT
<213>  Homo sapiens

<220>
<221>  MISC_FEATURE
<222>  (1)..(399)

<223>  Sequence of the human Sec62 protein

<400>  19

Met Ala Glu Arg Arg Arg His Lys Lys Arg Ile Gln Glu Val Gly Glu
1               5                   10                  15

Pro Ser Lys Glu Glu Lys Ala Val Ala Lys Tyr Leu Arg Phe Asn Cys
            20                  25                  30

Pro Thr Lys Ser Thr Asn Met Met Gly His Arg Val Asp Tyr Phe Ile
        35                  40                  45

Ala Ser Lys Ala Val Asp Cys Leu Leu Asp Ser Lys Trp Ala Lys Ala
    50                  55                  60

Lys Lys Gly Glu Glu Ala Leu Phe Thr Thr Arg Glu Ser Val Val Asp
65                  70                  75                  80

Tyr Cys Asn Arg Leu Leu Lys Lys Gln Phe Phe His Arg Ala Leu Lys
                85                  90                  95

Val Met Lys Met Lys Tyr Asp Lys Asp Ile Lys Lys Glu Lys Asp Lys
            100                 105                 110

Gly Lys Ala Glu Ser Gly Lys Glu Glu Asp Lys Lys Ser Lys Lys Glu
        115                 120                 125

Asn Ile Lys Asp Glu Lys Thr Lys Lys Glu Lys Glu Lys Lys Lys Asp
    130                 135                 140

Gly Glu Lys Glu Glu Ser Lys Lys Glu Glu Thr Pro Gly Thr Pro Lys
145                 150                 155                 160

Lys Lys Glu Thr Lys Lys Lys Phe Lys Leu Glu Pro His Asp Asp Gln
                165                 170                 175

Val Phe Leu Asp Gly Asn Glu Val Tyr Val Trp Ile Tyr Asp Pro Val
            180                 185                 190

His Phe Lys Thr Phe Val Met Gly Leu Ile Leu Val Ile Ala Val Ile
        195                 200                 205

Ala Ala Thr Leu Phe Pro Leu Trp Pro Ala Glu Met Arg Val Gly Val
    210                 215                 220

Tyr Tyr Leu Ser Val Gly Ala Gly Cys Phe Val Ala Ser Ile Leu Leu
225                 230                 235                 240

Leu Ala Val Asp Arg Cys Ile Leu Phe Leu Ile Ile Trp Leu Ile Thr
                245                 250                 255

Gly Gly Arg His His Phe Trp Phe Leu Pro Asn Leu Thr Ala Asp Val
            260                 265                 270

Gly Phe Ile Asp Ser Phe Arg Pro Leu Tyr Thr His Glu Tyr Lys Gly
        275                 280                 285

Pro Lys Ala Asp Leu Lys Lys Asp Glu Lys Ser Glu Thr Lys Lys Gln
    290                 295                 300

Gln Lys Ser Asp Ser Glu Glu Lys Ser Asp Ser Glu Lys Lys Glu Asp
305                 310                 315                 320

Glu Glu Gly Lys Val Gly Pro Gly Asn His Gly Thr Glu Gly Ser Gly
                325                 330                 335

Gly Glu Arg His Ser Asp Thr Asp Ser Asp Arg Arg Glu Asp Asp Arg
            340                 345                 350

Ser Gln His Ser Ser Gly Asn Gly Asn Asp Phe Glu Met Ile Thr Lys
        355                 360                 365

Glu Glu Leu Glu Gln Gln Thr Asp Gly Asp Cys Glu Glu Asp Glu Glu
    370                 375                 380

Glu Glu Asn Asp Gly Glu Thr Pro Lys Ser Ser His Glu Lys Ser
385                 390                 395

<210>  20
<211>  399
<212>  PRT
<213>  Homo sapiens

<220>
<221>  MISC_FEATURE
<222>  (308)..(308)
<223>  Mutation from D to A (D308A)

<400>  20

Met Ala Glu Arg Arg Arg His Lys Lys Arg Ile Gln Glu Val Gly Glu
1               5                   10                  15

Pro Ser Lys Glu Glu Lys Ala Val Ala Lys Tyr Leu Arg Phe Asn Cys
            20                  25                  30

Pro Thr Lys Ser Thr Asn Met Met Gly His Arg Val Asp Tyr Phe Ile

        35                  40                  45

Ala Ser Lys Ala Val Asp Cys Leu Leu Asp Ser Lys Trp Ala Lys Ala
    50                  55                  60

Lys Lys Gly Glu Glu Ala Leu Phe Thr Thr Arg Glu Ser Val Val Asp
65                  70                  75                  80

Tyr Cys Asn Arg Leu Leu Lys Lys Gln Phe Phe His Arg Ala Leu Lys
                85                  90                  95

Val Met Lys Met Lys Tyr Asp Lys Asp Ile Lys Lys Glu Lys Asp Lys
            100                 105                 110

Gly Lys Ala Glu Ser Gly Lys Glu Glu Asp Lys Lys Ser Lys Lys Glu
        115                 120                 125

Asn Ile Lys Asp Glu Lys Thr Lys Lys Glu Lys Glu Lys Lys Lys Asp
    130                 135                 140

Gly Glu Lys Glu Glu Ser Lys Lys Glu Glu Thr Pro Gly Thr Pro Lys
145                 150                 155                 160

Lys Lys Glu Thr Lys Lys Lys Phe Lys Leu Glu Pro His Asp Asp Gln
                165                 170                 175

Val Phe Leu Asp Gly Asn Glu Val Tyr Val Trp Ile Tyr Asp Pro Val
            180                 185                 190

His Phe Lys Thr Phe Val Met Gly Leu Ile Leu Val Ile Ala Val Ile
        195                 200                 205

Ala Ala Thr Leu Phe Pro Leu Trp Pro Ala Glu Met Arg Val Gly Val
    210                 215                 220

Tyr Tyr Leu Ser Val Gly Ala Gly Cys Phe Val Ala Ser Ile Leu Leu
225                 230                 235                 240

Leu Ala Val Asp Arg Cys Ile Leu Phe Leu Ile Ile Trp Leu Ile Thr
                245                 250                 255

Gly Gly Arg His His Phe Trp Phe Leu Pro Asn Leu Thr Ala Asp Val
            260                 265                 270

Gly Phe Ile Asp Ser Phe Arg Pro Leu Tyr Thr His Glu Tyr Lys Gly
        275                 280                 285

Pro Lys Ala Asp Leu Lys Lys Asp Glu Lys Ser Glu Thr Lys Lys Gln
    290                 295                 300

Gln Lys Ser Xaa Ser Glu Glu Lys Ser Asp Ser Glu Lys Lys Glu Asp
305                 310                 315                 320

Glu Glu Gly Lys Val Gly Pro Gly Asn His Gly Thr Glu Gly Ser Gly
                325                 330                 335

Gly Glu Arg His Ser Asp Thr Asp Ser Asp Arg Arg Glu Asp Asp Arg
            340                 345                 350

Ser Gln His Ser Ser Gly Asn Gly Asn Asp Phe Glu Met Ile Thr Lys
        355                 360                 365

Glu Glu Leu Glu Gln Gln Thr Asp Gly Asp Cys Glu Glu Asp Glu Glu
    370                 375                 380

Glu Glu Asn Asp Gly Glu Thr Pro Lys Ser Ser His Glu Lys Ser
385                 390                 395

## Description

The present invention relates to novel combinations of active ingredients for use in the prevention and/or treatment of tumors. The tumors treated by the composition according to the invention overexpress *SEC62* gene and overproduce Sec62 protein. The combination of active ingredients comprises at least a SERCA inhibitor and at least a Calmodulin antagonist.

### Background

Cancer is one of the most common deadly diseases [1] and the proportion of patients dying due to malignant diseases is increasing every year[2]. For example, lung cancer remains particularly in focus, with five-year survival rates below 20% [3] and therapeutic opportunities are particularly scarce for patients suffering from squamous cell carcinoma (SCC) of the lung [4].

Apoptosis is a process involving programmed cell death or "cellular suicide" and under physiological conditions it occurs after cell surface death receptors are occupied or activated by various genotoxic agents. This process leads to mitochondrial release of cytochrome C, which in turn activates the caspase enzymes that promote apoptosis.

Since apoptosis was first described in 1972 by Kerr et al. much knowledge has been accumulated about this important cellular process. Although a comprehensive understanding of apoptosis at the molecular level and cellular level is yet to be achieved, the knowledge accumulated thus far has led to the realization that because the process is genetically programmed, it may be susceptible to the effects of mutation and, therefore, may be involved in the pathogenesis of a variety of human diseases such as viral infections, autoimmune diseases and cancer . Based on this realization, it has been widely recognized that any therapeutic strategy aimed at specifically triggering apoptosis in diseased cells that suffer from disregulation of apoptosis (e.g. cancer) may deliver potentially promising therapies.

The endoplasmic reticulum (ER) stress response (ESR) consists of a set of adaptive pathways that can be triggered by disparate perturbations of normal ER function, such as accumulation of unfolded proteins, lipid or glycolipid imbalances, or changes in the ionic conditions of the ER lumen. The primary purpose of the ESR is to alleviate stressful disturbance and restore proper ER homeostasis; however, in the case of intense or persistent ER stress, these pathways will trigger programmed cell death/apoptosis. One of the central pro-survival regulators of the ESR is glucose-regulated protein 78 (GRP78/BiP), which has important roles in protein folding and assembly, in targeting misfolded protein for degradation, in ER Ca²⁺-binding, and in controlling the activation of trans-membrane ER stress sensors.. On the other hand, CCAAT/enhancer binding protein homologous transcription factor (CHOP/GADD153) and caspase 4 are critical executioners of the pro-apoptotic arm of the ESR .

The relevance of ER stress to tumor growth and survival is recognized. For example, one effective mode of triggering ER stress response is the inhibition of the sarcoplasmic/endoplasmic reticulum Ca²⁺-ATPase (SERCA), an intracellular membrane bound enzyme which sequesters cytosolic Ca²⁺-into its intracellular ER storage compartment. Inhibition of SERCA leads to the release of Ca²⁺-into the cytoplasm and resulting in activation of severe ER stress, leading to apoptosis.

Several inhibitors of SERCA have been described, to be used as a pro-apoptotic agent for cancer therapy. For instance, the most potent SERCA inhibitor, is the natural product thapsigargin. Another compound that was found to be a SERCA inhibitor is the anti-inflammatory drug celecoxib (Celebrex(R)) which is an inhibitor of cyclooxygenases-2 (COX-2) (Dannenberg, A J and Subbaramaiah, K, Cancer Cell 2003:4:431). Hydroquinone derivatives are also known as SERCA inhibitor, in particular 2,5-di-tert-buthyhydroquinine (Paula et al. J.bmc 17 (2009) 6613-6619). Paula et al. also reports other known SERCA inhibitors (paragraph 1. Introduction first column last paragraph), for examples cyclopiazonic acid, clotrimazole, thioronium benzene derivatives, tetrabromobisphenol, polyphenols and curcumin are mentioned. SERCA inhibitors are disclosed in US2011318837 and WO2010088450. US2003149995 disclosed a method for screening compounds that affect the activity of SERCA.

Sec 61 complex is a protein complex which has been identified to be a ER Ca2+ leak channel I a membrane protein insertase and a protein translocator. Calmodulin has been identified as limiting Ca(2+) leakage in a Ca(2+)-dependent manner by binding to an IQ motif in the cytosolic aminoterminus of Sec6lα.

Calmodulin is a calcium-binding messenger protein produced in all eukaryotic cells. CaM is a multifunctional intermediate messenger protein that transduces calcium signals by binding calcium ions and then modifying its interactions with various target proteins. Calmodulin is a small, highly conserved protein approximately 148 amino acids long (16706 Daltons). It contains four EF-hand motifs, each of which binds a Ca²⁺ ion. The protein has two approximately symmetrical globular domains (the N- and C-domain), separated by a flexible linker region. Calcium participates in an intracellular signaling system by acting as a diffusible second messenger to the initial stimuli.

Several compounds are known to be Calmodulin antagonist such as ophibolin A, Terodiline hydrochloride, Deltamethrin, trifluoperazine,- Zaldaride maleate, W-13 isomer hydrochloride (CAS # 88519-57-7); W5 (CAS # 61714-25-8), E6 Berbamine (CAS # 73885-53-7), N-(6-Aminohexyl)-5-chloro-1-naphthalenesulfonamide Hydrochloride; Calmidazolium Chloride (CAS 57265-65-3); Fluphenazine-N-2-chloroethane · 2HCl (CAS # 83016-35-7); CAS # 187937-24-2 (CAS # 187937-24-2); W-13 Isomer (CAS # 79127-24-5), Decyl Analog hydrochloride (CAS # 79127-24-5); Phenoxybenzamine (CAS # 63-92-3) and several others.

*SEC62* has been shown to be a new candidate oncogene that shows significant overexpression and elevated protein content in SCC[5]. Sec62 is an essential protein in yeast, where it is part of the Sec62/Sec63 sub-complex of the SEC complex and acts as a docking site for post-translational protein transport[6]. In mammals, studies have shown that Sec62 is associated with the heterotrimeric Sec61-complex and Sec63[7], and that Sec62 participates in the targeting and translocation of small pre-secretory proteins to the ER[8, 9]. Mammalian Sec62 is also reportedly able to interact with the ribosome, thereby regulating translation[10].

Elevated Sec62 protein content has been shown to be functionally linked to increased cell migration capability [11] and to reduced sensitivity to thapsigargin-induced ER stress [12] both of which are crucially regulated by the cytosolic and ER luminal calcium concentration[13-15].

Previous studies showed that Sec62 depletion by transfection with *SEC62* siRNA leads to inhibited cell migration and higher sensitivity to ER stress induced by calcium dysregulation [5, 11, 12]. Therefore, *SEC62* gene silencing seemed to provide a promising approach for cancer treatment, especially lung prostate and thyroid cancer, as such treatment could lead to reduced metastatic outspread of tumor cells as well as a higher cell sensitivity to chemotherapeutics working by ER stress induction.

However, despite intensive studies during the past decades [27-30], using RNA interference for the clinical treatment of human diseases remains unfeasible, mainly because of toxic side effects and major problems in achieving adequate concentrations in the target tissue [31].

There is therefore the need of a pharmaceutical treatment that is able to treat tumor overexpressing Sec62 protein both 1) by inhibiting the cell migration mechanism favored by the overproduction of Sec62 protein and 2) by counteracting the effect that the Sec62 protein overproduction has in reducing the effect of SERCA inhibitor used in the treatment of tumor.

### Summary of the invention

The present inventors have found that the administration of a Calmodulin antagonist in combination with SERCA inhibitor is useful in the treatment of tumors. The tumors are in particular characterized by the overexpression of the *SEC62* gene and hence by the overproduction of Sec62 protein.

Namely, the present inventors have found that the combined use of a SERCA inhibitor and a Calmodulin antagonist acts 1) on the inhibition of cell migration and 2) counteracts the reduced efficiency of SERCA inhibitor observed in the treatment of tumor overexpressing *SEC62* gene overproducing Sec62 protein, thereby providing an effective treatment of tumors overexpressing *SEC62* gene and hence overproducing Sec62 protein.

SERCA inhibitors such as thapsigargin induce tumoral cell apoptosis by inhibiting SERCA. Namely, the SERCA pump which transports the Ca²⁺ from the cytosol to the ER is inhibited. As a consequence an elevated concentration of Ca²⁺ in the cytosol and a lower concentration of Ca²⁺ in the ER is observed with a consequent induction of apoptosis. Calmodulin antagonist stimulates Ca²⁺ efflux from ER to cytosol via Sec61 (when the Sec61 channel is open) in the presence of SERCA inhibitors such as thapsigargin. In other words, Calmodulin antagonists are stimulating the Ca²⁺ leakage from ER to cytosol by acting directly on Calmodulin (and indirectly via Sec61 as Sec61 contains a Calmodulin binding site in the cytosolic N terminus of the mammalian Sec61 alpha subunit).

It has been further observed that when *SEC62* is overproduced there is an improvement in sealing of the Sec61 complex and therefore there is a reduction of the Ca²⁺ efflux from ER to cytosol. It has been observed that therefore an overexpression of Sec62 counteracts the action of the SERCA inhibitors such as thapsigargin in raising the cytosol Ca²⁺.

It has been surprisingly found by the present inventors that Calmodulin antagonists have also an effect on the Ca²⁺ leakage from ER to cytosol thereby overcoming the problem of the reduced efficacy of SERCA inhibitors in the presence of Sec62 overexpression. This is particularly surprising because there is no indication in the art that Calmodulin antagonists may counteract the reduction of efflux of Ca²⁺ from ER to cytosol due to Sec62 overexpression.

The present inventors have found that a strong inhibition of cell migration in different human cancer cells is achieved after Sec62 depletion by transfection with *SEC62* siRNA [5, 11]. Moreover, silencing of the *SEC62* gene markedly increased the cells' sensitivity to ER stress induced by dysregulation of cellular calcium homeostasis, as shown by the more pronounced growth inhibition of *SEC62*-depleted cells after treatment with the SERCA inhibitor thapsigargin compared to control cells [5, 12].

These results indicated that Sec62 plays a crucial role in cell migration and ER stress response, particularly in cancer cells. However, the molecular mechanisms these phenomena are based on are not understood and known, since the function of Sec62 is only partially understood even under physiological conditions.

Moreover, although a clear indicium that a Sec62 inhibitor would be useful in inhibiting cell migration and in increasing cell sensitivity to ER stress induced by dysregulation of cellular calcium homeostasis, so far no inhibitors of Sec62 protein are known.

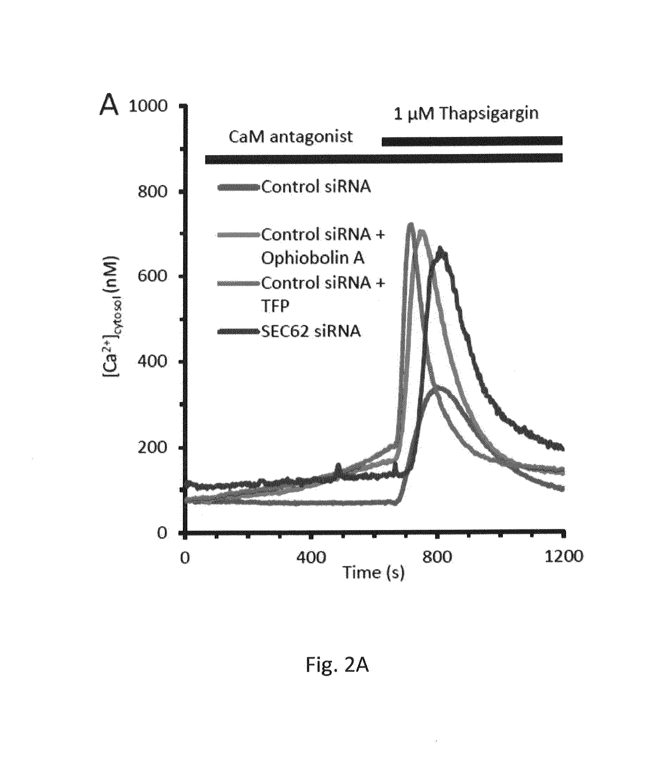

The present inventors have found that Sec62 depletion by siRNA transfection and the treatment of the cells with Calmodulin antagonists resulted in very similar changes of basal cellular calcium level. In addition they have found that the administration of a Calmodulin antagonist results in an increased of cytosolic calcium concentration after thapsigargin treatment (a SERCA inhibitor) (Figure 2A).

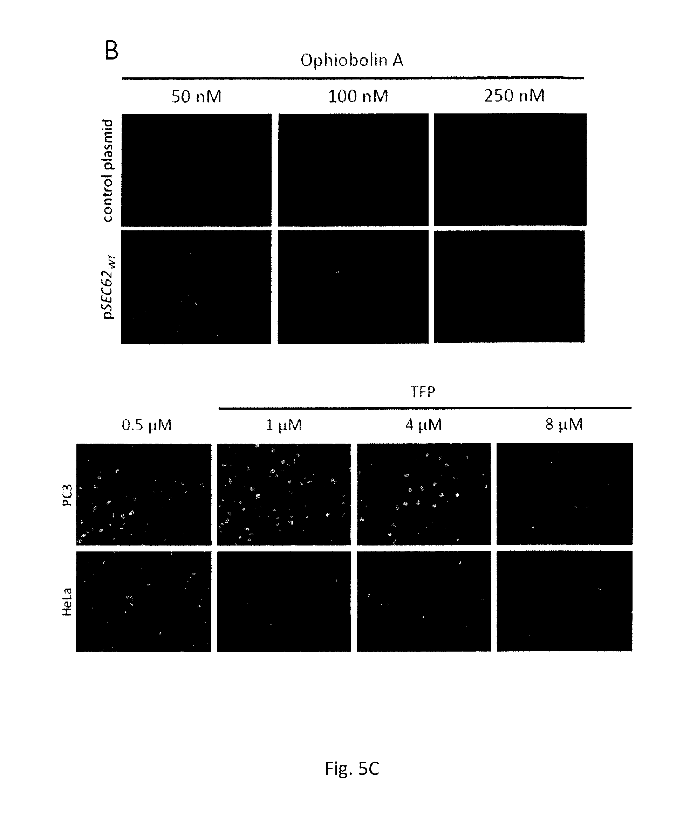

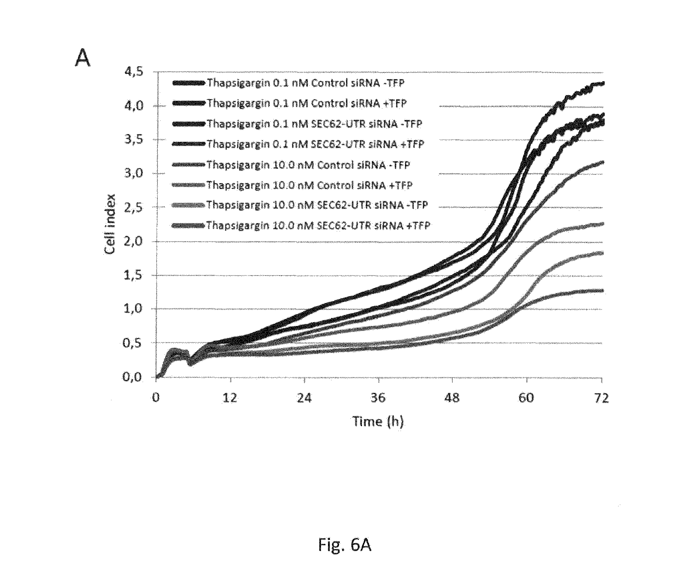

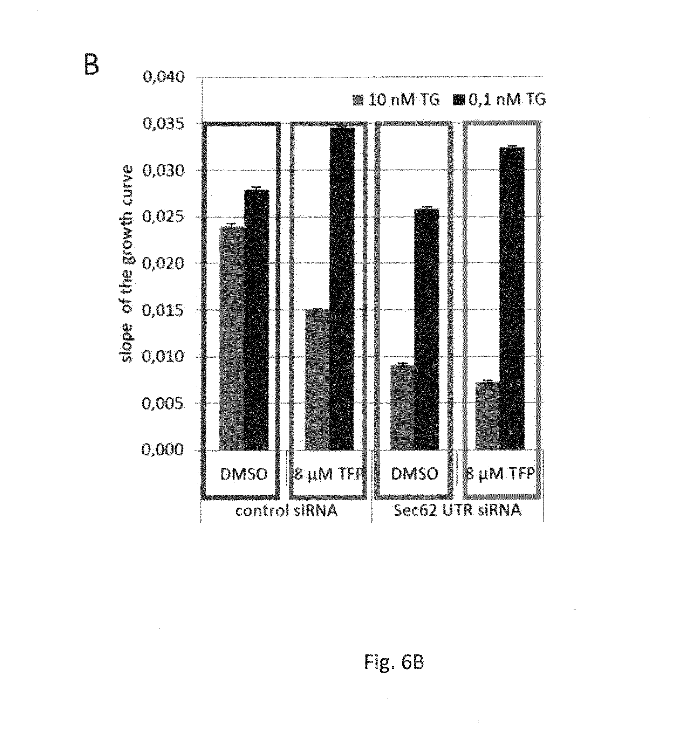

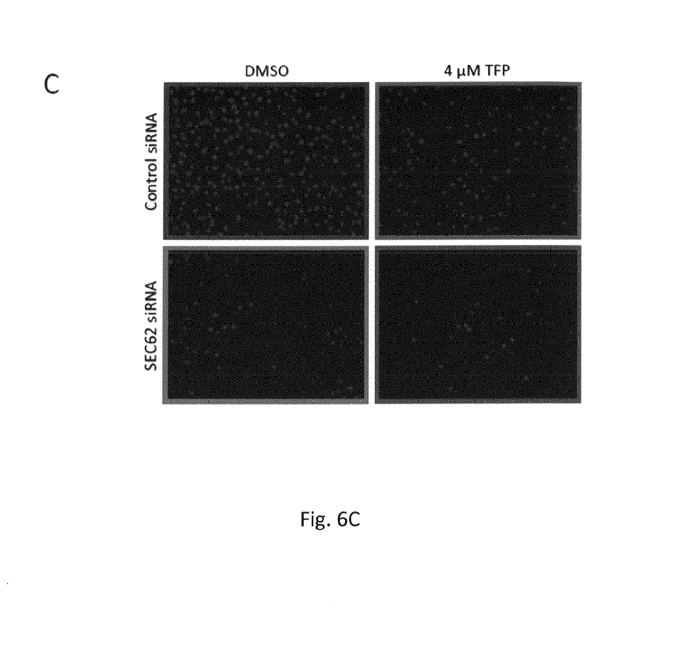

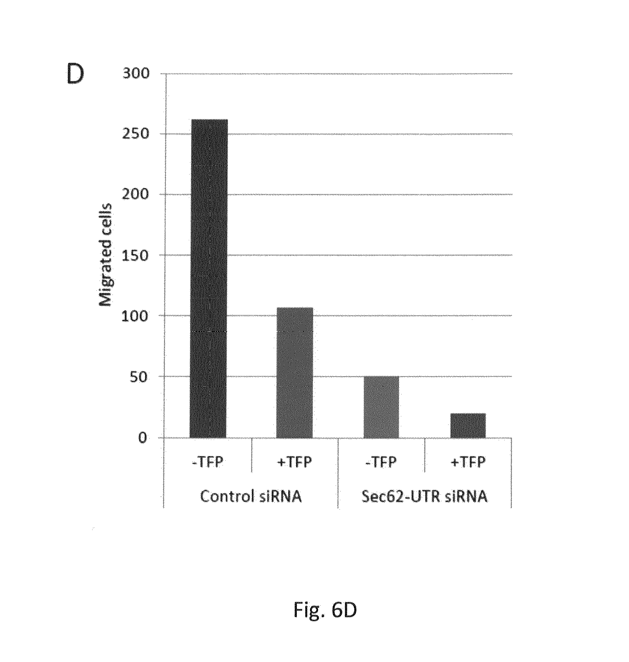

The present inventors also found that the treatment of different human cancer cells with Calmodulin antagonists leads to the same cellular phenotypes as observed after silencing the *SEC62* gene, namely inhibited cell migration and markedly higher cell sensitivity to thapsigargin-induced ER stress (Figure 6A and B). The crucial role of Sec62 in cellular calcium homeostasis is further supported by the observed dditive action of treatment with *SEC62* siRNA and Calmodulin antagonists regarding the sensitivity to thapsigargin-induced ER stress (Figure 6), as well as by the rescue of cell migration by *SEC62* overexpression in cells pretreated with Calmodulin antagonists (Figure 5B).

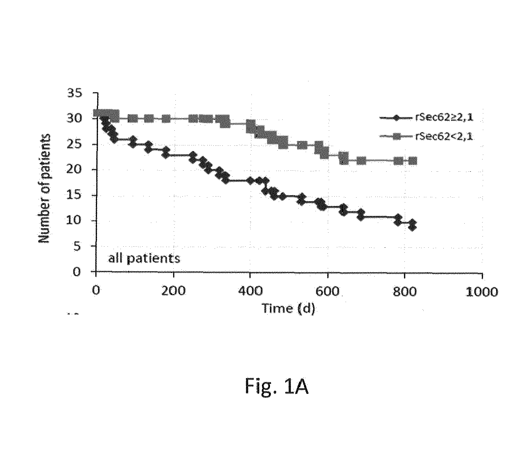

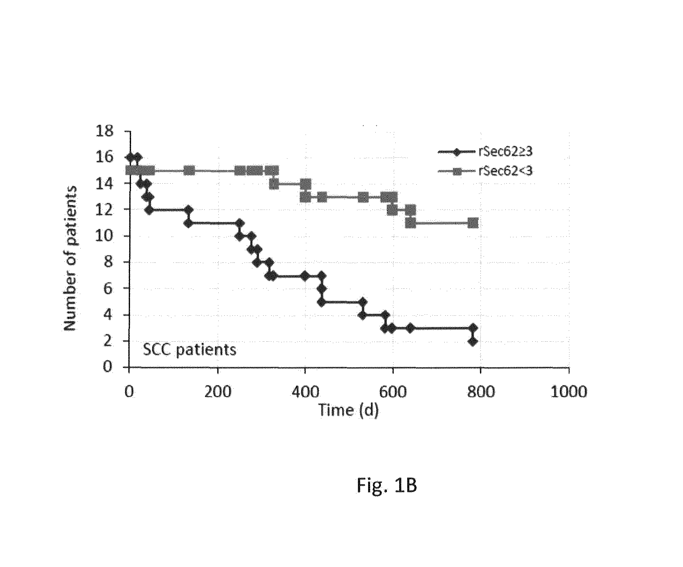

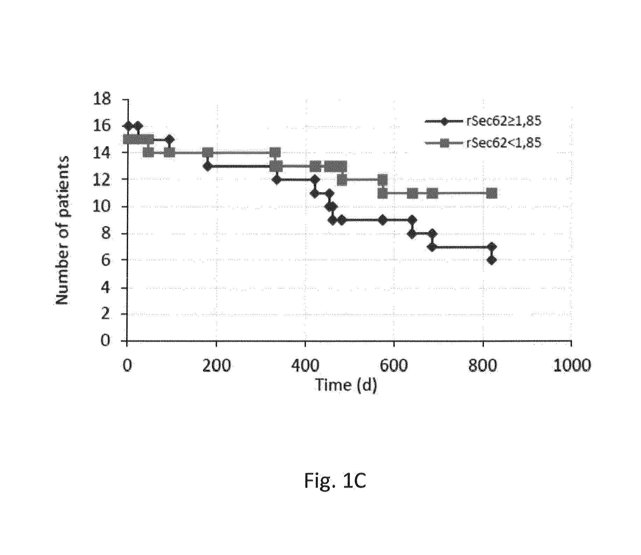

**Figure 1****. Sec62 protein concentration in cancer tissue represents a prognostic marker in NSCLC patients.** The Sec62 concentration in cancerous and tumor-free lung tissue of 70 NSCLC patients by western blot were analyzed. Patients were divided into two groups based on rSec62 value, and survival analyses were performed using the Kaplan-Meier method and log-rank-test. **Fig. 1A****,** Patients with rSec62 < 2.1 showed significantly longer survival compared to those with rSec62 ≥ 2.1 (*P* < 0.001). **Fig. 1B****,** The survival benefit of patients with a low Sec62 protein level in the lung cancer tissue was significant in SCC patients analyzed separately (*P* < 0.001), **Fig. 1C****,** but not when AC patients were analyzed separately (*P* = 0.051).

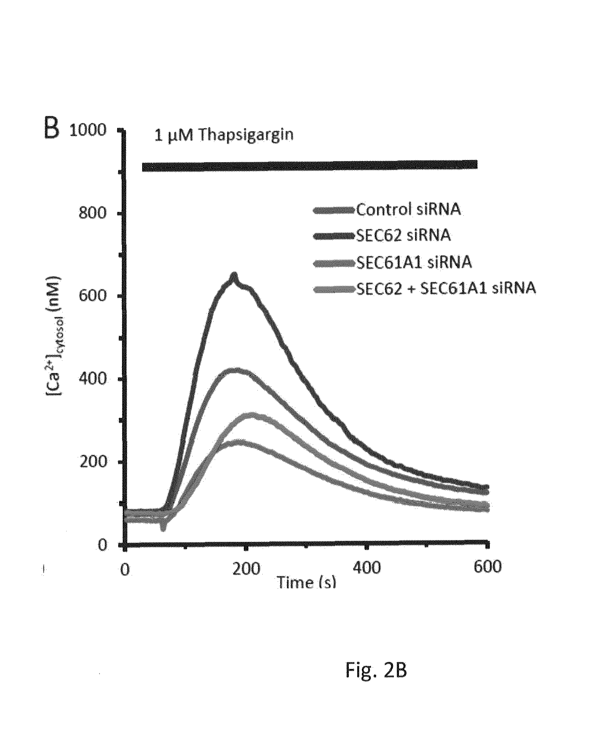

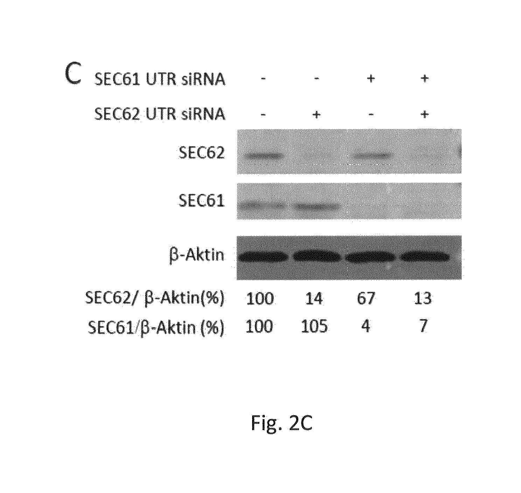

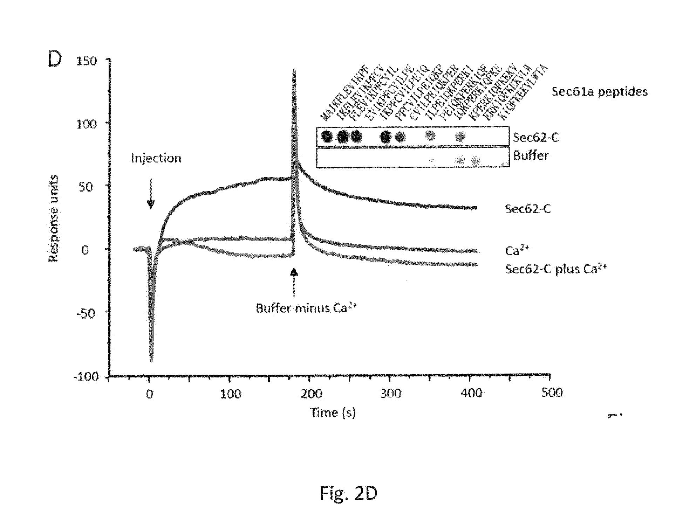

**Figure 2****. Comparison of cytosolic calcium concentrations after *SEC62* silencing and treatment with the Calmodulin antagonists.** **Fig. 2A**, HeLa cells were seeded on glass-slides in 6-cm dishes. After 24 and 48 h, cells were transfected with *SEC62* siRNA or control siRNA; 48 h after the second transfection, cells were loaded with FURA2-AM dye. After 45 min, cells were used for calcium imaging. After a 60-s incubation in calcium-free buffer, control siRNA transfected cells were treated with buffer alone, buffer + ophiobolin A (100 µM), or buffer + TFP (10 µM) for 600 s; subsequently, 1 µM thapsigargin was added. The curves shown in the diagram represent the mean values of cytosolic calcium concentration of 182 cells (control siRNA + buffer), 325 cells (control siRNA + ophiobolin A), or 198 cells. **Fig. 2B****,** HeLa cells were seeded on glass-slides in 6-cm dishes. After 24 and 48 h, cells were transfected with *SEC62* siRNA, *SEC61A1* siRNA, a combination of both siRNAs, or control siRNA; cell were loaded with FURA2AM dye and pre-incubated in calcium-free buffer as described in A, than 1 µM thapsigargin was added. The curves shown in the diagram represent the mean values of cytosolic calcium concentration of 547 cells (control siRNA), 353 cells (*SEC62* siRNA), 495 cells (*SEC61A1* siRNA) or 395 cells (*SEC62* + *SEC61A1* siRNA) taken from three independent experiments. **Fig. 2C****,** Silencing efficiency in the experiment described in B was determined by western blot analysis. The numbers given in the figure represent the mean value of the three independent experiments, the western blots show a representative result. D, Surface plasmon resonance spectroscopy was performed on a CM5 sensor chip with Sec61a N-terminal peptide coupled to the measuring cell and TRAM N-terminal peptide coupled to the control cell. Purified Sec62-C-His (1 µM) was passed over both cells in buffer minus Ca²⁺ or the same buffer containing 2 mM Ca²⁺. The Ca²⁺ containing buffer alone was used as a control. Response units are shown as a difference between measuring- and control cell. The insert shows a peptide spot binding assay. Here 13 peptides scanning the N-terminus of Sec61a were synthesized on a cellulose membrane and incubated with purified Sec62-C-His (1 µM) in binding buffer. Bound protein was detected using anti-His-POD coupled antibody and visualized with a lumi-imaging system. We note that the slight reduction of Sec62 protein content after *SEC61A1* siRNA treatment has to be interpreted as an unspecific variation as, in general, Sec61a protein depletion does not affect Sec62 protein content [9].

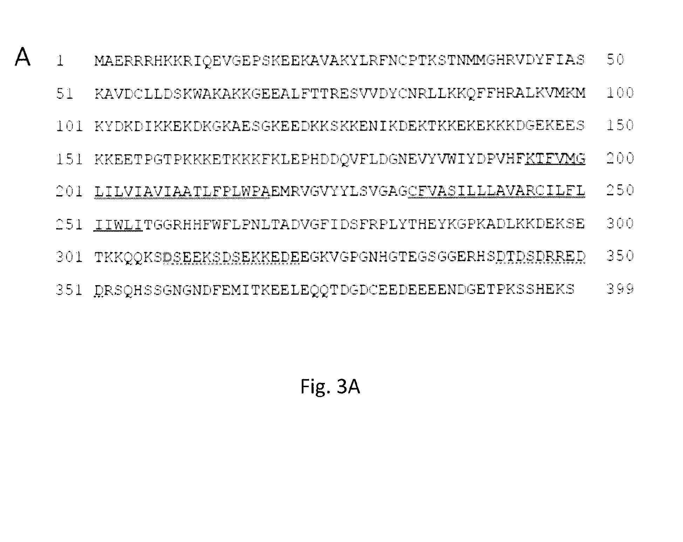

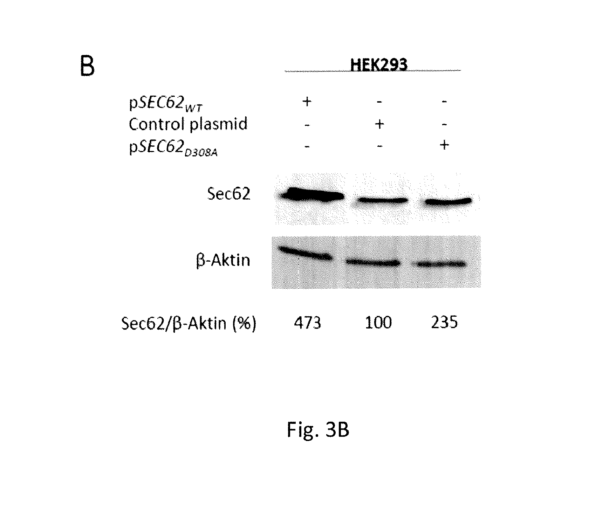

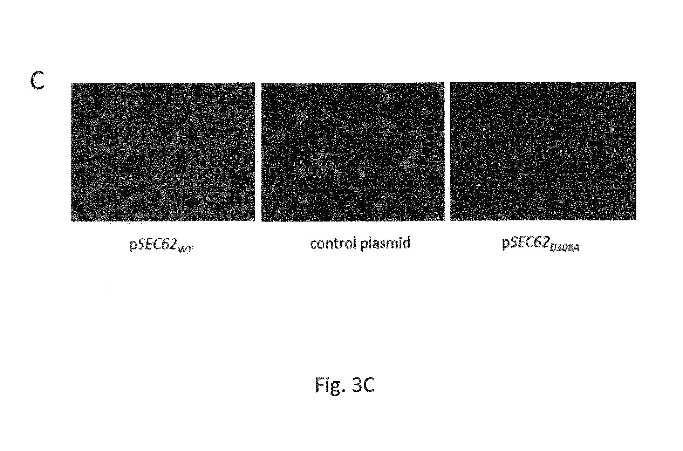

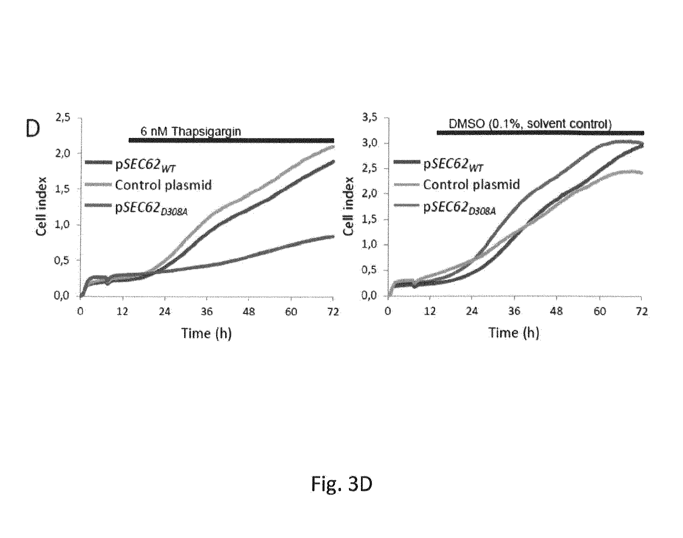

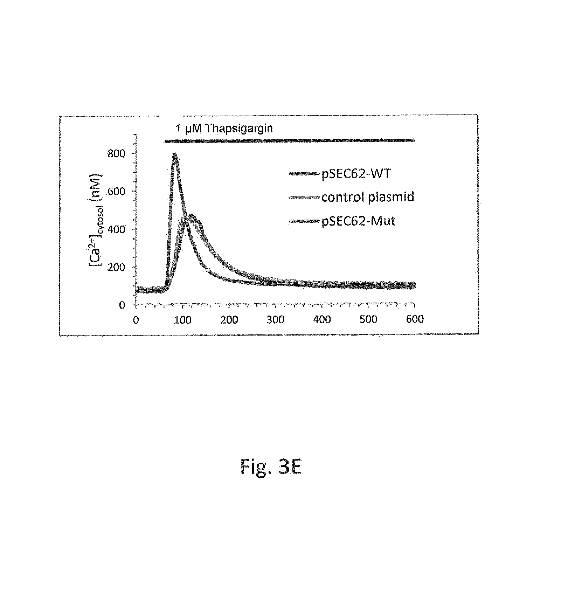

**Figure 3****. A mutation in Sec62's putative EF-hand motif dominant-negatively effects cell-migration, -growth, and ER calcium efflux.** **Fig.3A****,** Sequence of the human Sec62 protein. Transmembrane domains 1 and 2 are underlined with a solid line. The predicted EF-hand motif is underlined with a dotted line. In the plasmid-encoded *SEC62_{D308A}* gene, amino acid D308 (shown in red) was replaced by an alanine. **Fig.3B****,** Sec62 protein content was analyzed in the stably transfected HEK293 cell lines by western blot analysis. **Fig.3C****,** HEK293 cells stably transfected with either pIRES-GFP-*SEC62*-WT, pIRES-GFP-*SEC62_{D308A},* or pIRES-GFP (control plasmid) were seeded in normal growth medium without FBS and in the top chamber of the BD-Falcon Fluoroblok migration system (24-well format). The lower chamber contained the same medium with 10% FBS as an attractant. After 72 h, migrated cells were fixed with methanol and DAPI stained; migration was analyzed by fluorescence microscopy using a 10-fold objective magnification. **Fig.3D****,** Stably transfected HEK293 cells as described in B, (5 × 10³) were seeded in a 96-well ePlate and growth was examined in the xCELLigence RTCA system. After 300 min, 6 nM thapsigargin (left panel) or 0.1% DMSO (solvent control, right panel) was added to each well. All samples were measured in triplicates. E, Stably transfected HEK293 cells as described in B, were seeded on glass-slides in 6-cm dishes and loaded with FURA2-AM dye; 45 min later, they were used for calcium imaging. After 60 s incubation with EGTA buffer, cells were treated with 1 µM thapsigargin. The curves shown in the diagram represent the mean values of cytosolic calcium concentration of 158 cells (p*SEC62*-WT), 159 cells (p*SEC62_{D308A}*)*,* or 160 cells (control plasmid) cells.

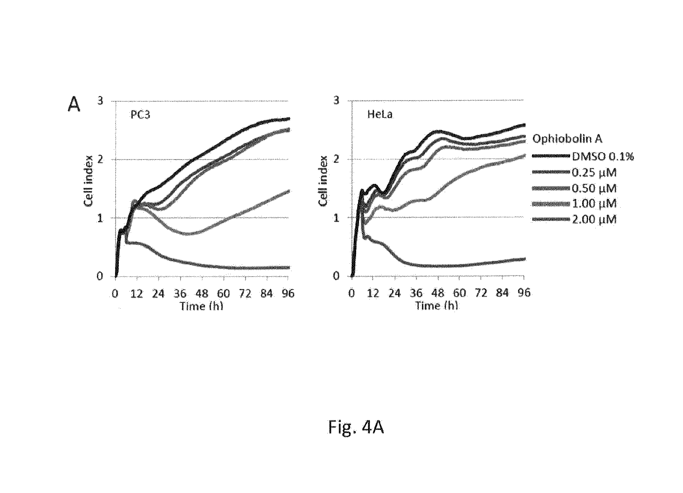

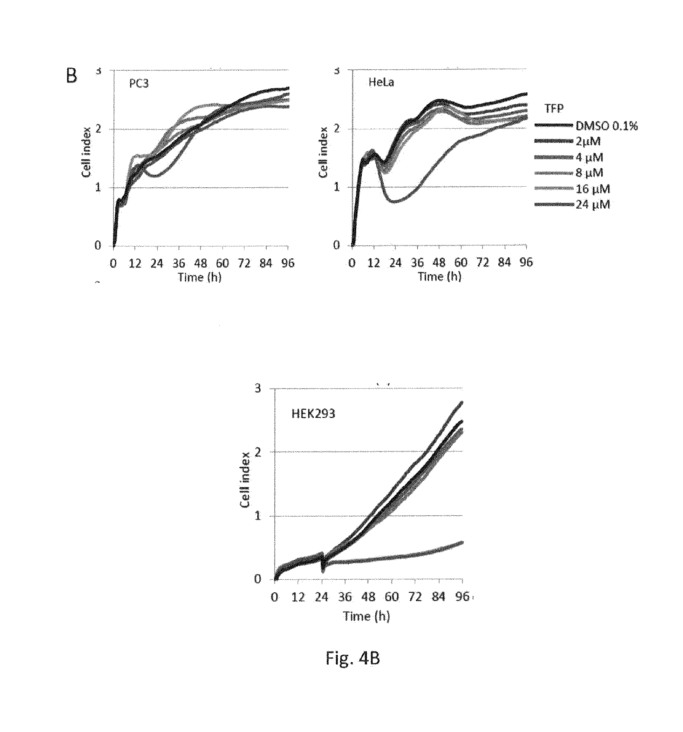

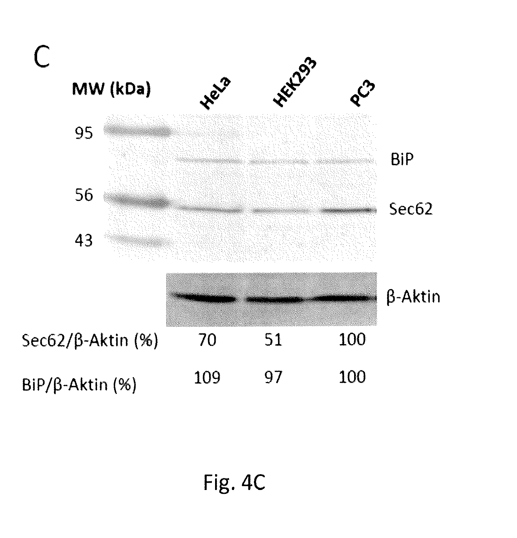

**Figure 4****. Reduced *SEC62* expression correlates with slightly increased sensitivity to TFP compared to in PC3 cells.** **Fig. 4A****,** Effect of ophiobolin A treatment on the cell growth of PC3 or HeLa cells. PC3 or HeLa cells (1 × 10⁴) were seeded in a 96-well ePlate and growth was examined in the xCELLigence RTCA system. After 330 min, cells were treated with buffer alone or buffer + ophiobolin A in the concentrations mentioned in the figure. All samples were measured in triplicates. The cell index was normalized to the time point of cell treatment (330 min). **Fig. 4B****,** The same analysis as described in A was performed on PC3, HeLa, and HEK293 cells after treatment with TFP in the concentrations mentioned in the figure. **Fig. 4C****,** Quantification of the ER proteins Sec62 and BiP by western blot analysis.

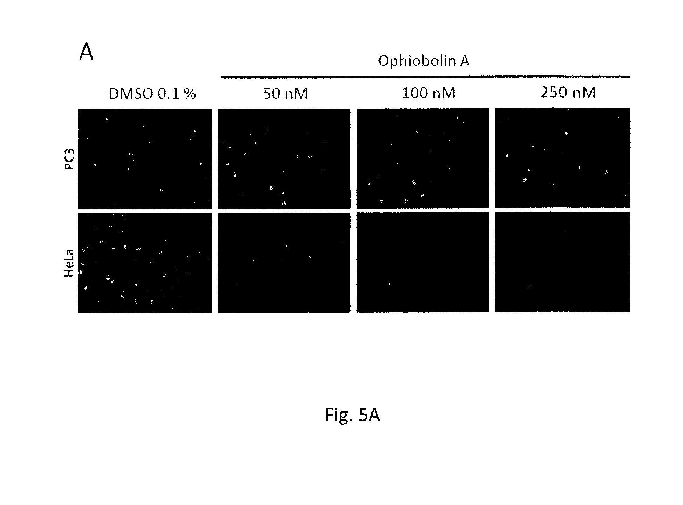

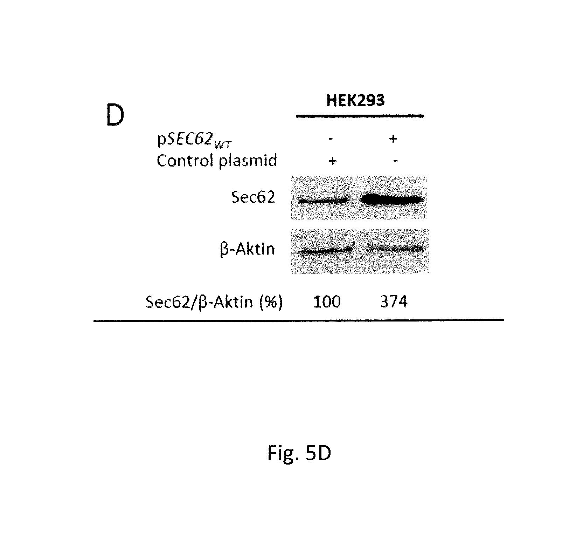

**Figure 5****. Calmodulin antagonist treatment effects cell migration which can be rescued by *SEC62* overexpression.** **Fig.5A**, PC3- or HeLa cells were seeded in normal growth medium without FBS and supplemented with either ophiobolin A, TFP or DMSO as a control in the mentioned concentrations in the top chamber of the BD-Falcon Fluoroblok migration system (24-well format). The upper chambers were settled into the lower chamber, containing the same medium with 10% FBS as an attractant. After 72 h (PC3) or 24 h (HeLa), migrated cells were fixed with methanol and DAPI stained; subsequently, migration was analyzed by fluorescence microscopy. **Fig.5B****,** HEK293 cells stably transfected with a plasmid encoding for wild-type *SEC62* (p*SEC62*-WT) or with the respective control plasmid were seeded in normal growth medium without FBS and supplemented with TPA (10 nM) and ophiobolin A in the indicated concentrations in the top chamber of the BD-Falcon Fluoroblok migration system (24-well format). The upper chambers were settled into the lower chamber, containing the same medium with 10% FBS as an attractant. After 72 h, migrated cells were fixed with methanol and DAPI stained; subsequently, migration was analyzed by fluorescence microscopy. **Fig.5C****,** The same experiments described in B, where performed in the presence of TFP instead of ophiobolin A in the indicated concentrations. **Fig.5D****,** Sec62 protein content was analyzed in the stably transfected HEK293 cell lines by western blot analysis.

**Figure 6****. *SEC62* silencing and TFP treatment additively effect cell growth and migration in PC3 cells.** **Fig. 6A**, Cells were seeded in 6-cm dishes and transfected 24 h and 48 h after seeding with *SEC62* siRNA or control siRNA; 24 h after the second transfection, 5 × 10³ PC3 cells were seeded in a 96-well ePlate and growth was examined in the xCELLigence RTCA system. After 300 min, cells were treated with 10 nM thapsigargin or 0.1 nM thapsigargin, and DMSO (0.1%, solvent control) or TFP (8 µM). All samples were measured in triplicates. **Fig. 6B****,** The slope of the growth curves shown in A between 8-72 h was calculated using the RTCA software. The error-bars indicate the standard deviation of the triplicates. **Fig. 6c****,** Cells were treated with *SEC62* siRNA or control siRNA as described in A. At 24 h after the second transfection, cells were seeded in normal growth medium without FBS and supplemented with either 4 µM TFP or 0.1% DMSO as a control in the top chamber of the BD-Falcon Fluoroblok migration system (24-well format). The lower chamber contained the same medium with 10% FBS as an attractant. After 72 h, migrated PC3 cells were fixed with methanol and DAPI stained; subsequently, migration was analyzed by fluorescence microscopy. **Fig. 6D****,** Migrated cells from C were automatically counted by using the NIS-Elements AR Software (Nikon).

### Detailed description of the invention.

The present invention is directed to a combination of active ingredients comprising at least a SERCA inhibitor compound in combination with at least a Calmodulin antagonist compound for use in the treatment of a tumor. The tumor is characterized by the overexpression of *SEC62* gene and hence by the overproduction of Sec62 protein.

"Overexpression of *SEC62* gene" is defined as an excessive expression of *SEC62* gene. *SEC62* gene overexpression may be observed and measured in a tumor histological tissue. The overexpression is measured with respect to the expression of *SEC62* gene relative to tumor free histological tissue. The tumor histological tissue and the tumor free histological tissue for comparison of determination of overexpression of *SEC62* gene are preferably from the same patient.

The overexpression of *SEC62* gene may be measured for example by measuring the *SEC62* mRNA, preferably the *SEC62* mRNA is from in a tumor histological tissue of a patient which is suspected to be affected by a tumor that overexpresses *SEC62* gene. The *SEC62* mRNA of tumor free histological tissue is measured as well. The quantitative values obtained are than compared to determine whether the *SEC62* gene is overexpressed in the tumor histological tissue. Preferably, the tumor histological tissue and the tumor free histological tissue are from the same patient. Preferably, it is considered that *SEC62* mRNA is overexpressed when its content in the tumor histological tissue is ≥ 1.5 fold with respect to *SEC62* mRNA content in tumor free histological tissue, preferably the tumor histological tissue and the tumor free histological tissue are from the same patient. Preferably, the mRNA content is ≥ 1.5 fold when measured with real time PCR. Even more, preferably it is considered that *SEC62* mRNA is overexpressed when its content in the tumor histological tissue is ≥ 2 fold with respect to *SEC62* mRNA content in tumor free histological tissue, preferably the tumor histological tissue and the tumor free histological tissue are from the same patient.

Methods of quantitatively measuring the overexpression of a gene are commonly known in the art. For example, Northern blotting, real time PCT (RT-PCR), RNA array or in situ hybridization (ISU) etc. are known methods. These methods are used also for the quantitative measurement of the *SEC62* gene overexpression. In particular, according to the present invention, a tumor overexpresses the *SEC62* gene if by the analysis of mRNA content in a tumor histological tissue, the analysis reveals a ≥ 1.5 fold, preferably a ≥ 2 fold, increase in *SEC62* mRNA expression relative to tumor free histological tissue of the same patient. The preferred method to measure *SEC62* mRNA is RT PCR.

In the present invention, alternatively or in combination with the measurement of mRNA, the overproduction of Sec62 protein can be measured. It is known to the skilled person that the overexpression of *SEC62* gene in a tumor histological tissue results in an overproduction of the Sec62 protein. Hence either or both the overexpression of *SEC62* gene and/or overproduction of Sec62 protein can be measured to determine the tumor to be treated according to the present invention.

"Overproduction of Sec62 protein" is defined as an excessive production of Sec62 protein encoded by the *SEC62* gene. Sec62 protein overproduction may be observed and measured in a tumor histological tissue. The overproduction is measured with respect to the production of Sec62 protein relative to tumor free histological tissue. The tumor histological tissue and the tumor free histological tissue for comparison of determination of overproduction of Sec62 protein are preferably from the same patient.

The overproduction of Sec62 protein may be measured for example by measuring the Sec62 protein, preferably Sec62 protein is from in a tumor histological tissue of a patient which is suspected to be affected by a tumor that overproduce Sec62 protein. Sec62 protein of tumor free histological tissue is measured as well. The quantitative values obtained are than compared to determine whether the Sec62 protein is overproduced in the tumor histological tissue. Preferably, the tumor histological tissue and the tumor free histological tissue are from the same patient. Preferably, it is considered that Sec62 protein is overproduced when its content in the tumor histological tissue is ≥ 1.5 fold, preferably a ≥ 2 fold, with respect to the Sec62 protein content in tumor free histological tissue, preferably the tumor histological tissue and the tumor free histological tissue are from the same patient. Preferably the protein content is measured with western blot.

Methods of quantitatively measuring the overproduction of a protein are commonly known in the art. For example western blot, histochemistry method such as immunohistochemistry or protein arrays etc. methods can be used. These methods are used also for the quantitative measurement of the Sec62 protein overproduction. In particular, according to the present invention, a tumor overproduces Sec62 protein if by the analysis of Sec62 protein content in a tumor histological tissue, the analysis reveals a ≥ 1.5 fold, preferably a ≥ 2 fold increase in Sec62 protein relative to tumor free histological tissue of the same patient. The preferred method to measure Sec62 protein is western blot.

Hence, the present invention is directed to a SERCA inhibitor in combination with Calmodulin antagonist for use in the treatment of tumor wherein the tumor is defined as a tumor that overexpresses *SEC62* gene and/or a tumor that overproduces sec62 protein. The skilled person understands that the overproduction of Sec62 protein is the result of overexpression of *SEC62* gene.

Hence, the present invention is directed to a SERCA inhibitor in combination with Calmodulin antagonist for use in the treatment of tumor wherein the tumor is defined as a tumor that overexpresses *SEC62* gene or a tumor that overproduces Sec62 protein and wherein the overexpression of *SEC62* gene is measured by the analysis of mRNA content in a tumor histological tissue and the analysis reveals a more than 1.5 fold increase in Sec62 expression relative to tumor free histological tissue preferably of the same patient and/or wherein the overproduction of Sec62 protein is measured by the analysis of Sec62 protein level in a tumor cell and the analysis reveal a more than 1.5 fold increase in Sec62 protein level relative to tumor free tissue preferably of the same patient.

Hence, the present invention is directed to a combination of at least SERCA inhibitor and at least a Calmodulin antagonist for use according to the present invention wherein the *SEC62* mRNA is measured with RT-PCR or with in situ hybridization or with RNA array and the Sec62 protein is measured with Western Blot, histochemistry method or protein arrays.

The SERCA inhibitor in combination with a Calmodulin antagonist for use according to the present invention is any compound suitable for inhibiting the SERCA activity. SERCA inhibitors are known in the art. The SERCA inhibitor compound according to the present invention may be selected from sesquiterpene lactones and derivative thereof (compounds built from 3 isoprene units and containing a lactone ring), such as thapsigargin and prodrug thereof such as 8-O-(12Aminododecanoyl)-8-O debutanoylthapsigargin (G202) and the peptide prodrug L12ADT and, artemisinin; hydroquinone based compounds such as 2,5-di(tert-butyl)hydroquinone (BHQ); 1,3-dibromo-2,4,6-tris (methyl-isothio-uronium) benzene, celecoxib cyclopiazonic acid; clotrimazole, thioronium benzene derivatives, tetrabromobisphenol, polyphenols and curcumin.

Thapsigargin and the prodrug L12ADT have formulae

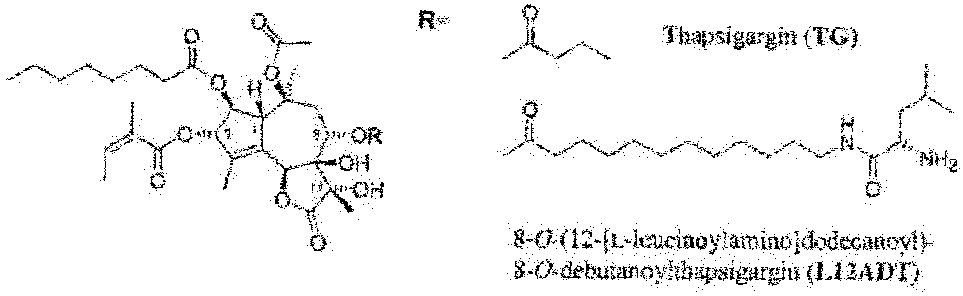

Thapsigargin and prodrug thereof, in particular G202 and L12ADT prodrug are preferred SERCA inhibitors according to the invention.

The Calmodulin antagonist according the present invention is any compound suitable for antagonizing Calmodulin. Calmodulin antagonists are known in the art. A Calmodulin antagonist according to the invention may be selected from trifluoperazine, Ophobolin A, the compound of formula 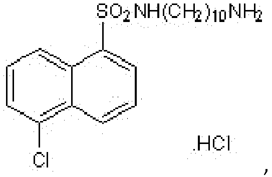 the compound of formula 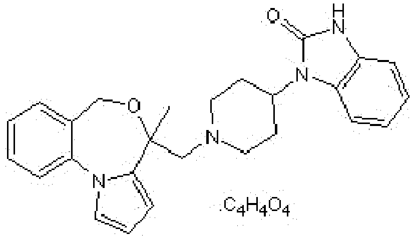 (also known as CGS 9343B and having CAS number: CAS 109826-27-9), Zaldaride maleate,
the compound of formula 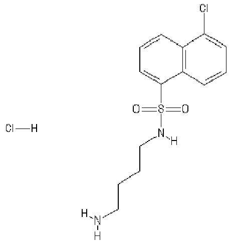 (also known as W-13 isomer hydrochloride (CAS # 88519-57-7));
the compound of formula 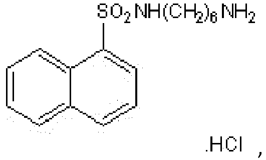 the compound of formula 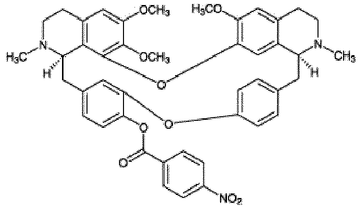 (also known as E6 Berbamine (CAS # 73885-53-7)), N-(6-Aminohexyl)-5-chloro-1-naphthalenesulfonamide Hydrochloride; Calmidazolium Chloride (CAS 57265-65-3); the compound of formula 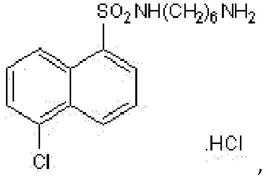 Fluphenazine-N-2-chloroethane · 2HCl (CAS # 83016-35-7),
the compound of formula 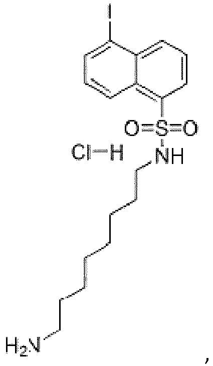 the compound of formula 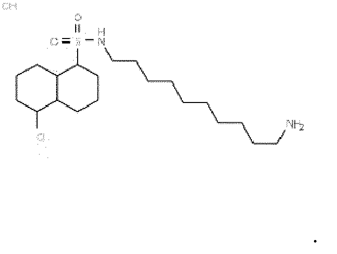 the compound of formula

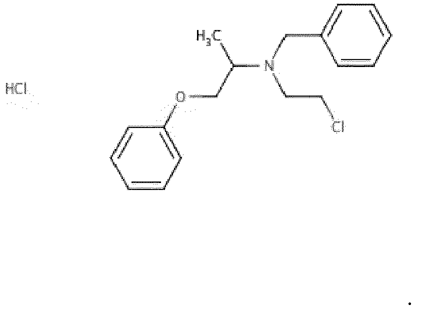

Preferred Calmodulin antagonists according to the invention are trifluoperazine and Ophiobolin A.

The present inventors have identified a group of tumors, namely the tumors that overexpress *SEC62* gene and hence overproduce Sec62 protein. The tumors treated by the combinations of the inventions are tumors that overexpress *SEC62* gene and hence overproduce Sec62 protein. Said tumors are for examples non-small lung cell lung cancer, tyroid cancer, prostate cancer, head and neck cancer, multiple myeloma and other. The present inventors have found that there are tumors of the same kind that do or do not overexpress *SEC62* gene and hence do or do not overproduce Sec62 protein. For example, it has been seen by the present inventors that only about half of the prostate cancer shown an overexpression of *SEC62* gene (Greiner et al. The prostate 71:1074-1083 (2011). Hence, the present "prostate cancer" as used herein is not any prostate cancer but a subgroup of prostate cancer, specifically the prostate cancer that overexpresses *SEC62* gene and hence overproduces Sec62 protein. The same applies to other tumors. Hence, the present invention is directed to the treatment of a subgroup of non small lung cell lung cancer, a subgroup of tyroid cancer, a subgroup of prostate cancer, a subgroup of head and neck cancer, a subgroup of multiple myeloma and a subgroup of tumors wherein said subgroup of the specified tumor overexpresses *SEC62* gene and hence overproduces Sec62 protein.

In an embodiment, the SERCA inhibitor in combination with a Calmodulin antagonist for use in the treatment of tumor wherein the tumors are as defined according to the present invention wherein the SERCA inhibitor and the Calmodulin antagonist are administered simultaneously or sequentially or separately.

By simultaneous therapeutic use, within the meaning of the present invention is meant a administration of at least a SERCA inhibitor and a Calmodulin antagonist by the same route and at the same time or at approximately the same time.

By separate use, within the meaning of the present invention is meant in particular an administration of at least a SERCA inhibitor and a Calmodulin antagonist at the same time or at substantially the same time by different routes.

By sequential therapeutic use is meant administration of at least a SERCA inhibitor and a Calmodulin antagonist at different times, the administration route being identical or different. More particularly by an administration method is meant a method according to which the whole administration of one of the active ingredients is carried out before administration of the other or others commences. It is thus possible to administer one of the active ingredients 1-3 hours before administering the other active ingredient or ingredients. Preferably 1 hour. There is no simultaneous treatment in this case. Hence, the SERCA inhibitor in combination with a Calmodulin antagonist for use according to the present invention are administered sequentially within an interval of 1 to 3 hours preferably, 1 hour.

The pharmaceutical formulation of a combination or combined preparation of the invention is dependent upon the administration route, in accordance with the usual practice in the field.

In a combination or combined preparation of the invention, said at least one SERCA inhibitor and a Calmodulin antagonist can e.g., be formulated in a form suitable for parenteral, trans-mucosal, enteral or topical administration, advantageously for intravenous administration. Said at least one SERCA inhibitor and a Calmodulin antagonist may be formulated for different administration routes for example, in a form suitable for parenteral administration for said at least one SERCA Inhibitor or a Calmodulin antagonist, and in a form suitable for trans-mucosal administration for said at least SERCA Inhibitor or a Calmodulin antagonist. Alternatively, they can be both formulated for the same administration route (for example, both in a form suitable for parenteral/intravenous administration).

A preferred parenteral administration is the intravenous administration.

In an embodiment of the invention, a combination or combined preparation of the invention is formulated in a form suitable for, or is intended for systemic or intra-tumoral administration.

The determination of the dose regimen of said at least SERCA Inhibitor and a Calmodulin antagonist inhibitor is determined by the person of ordinary skill in the art so as to achieve the desired effect, notably the treatment of the tumor of the invention.

The dose regimen, more particularly the dose amount (or dose per administration) of said at least one SERCA inhibitor and a Calmodulin antagonist, is advantageously such that said at least a SERCA inhibitor and a Calmodulin antagonist exert an advantageous effect, in the treatment of tumors according to the invention. The effect may also be intended as due to the counteraction of the Calmodulin antagonist on the reduce efflux of Ca²⁺ from ER to cytosol due to Sec62 protein overproduction. As already explained, the overproduction of Sec62 protein reduces the efficiency of SERCA inhibitors in the treatment of those tumors that overexpress *SEC62* gene. In addition, the Sec62 protein overproduction stimulates cell migration which is an undesirable effect in tumor treatment. Cell migration is inhibited by the Calmodulin antagonist, thereby leading to an improved treatment of the tumors of the invention.

For example, at a fixed dose amount of said at least SERCA inhibitor, the dose amount of said at least one Calmodulin antagonist can be increased up to the point where said advantageous effect is observed and/or the frequency of administration of each dose of said at least one Calmodulin antagonist can be increased up to said advantageous point. Hence, the dose of the Calmodulin antagonist can be increased till no negative effect of the overproduction of Sec62 protein is observed on the SERCA inhibitor and an effect on the inhibition of tumor cell migration is observed.

For example, the dose regimen, more particularly the dose amount of said at least one SERCA inhibitor and a Calmodulin antagonist is such that said at least one Calmodulin antagonist increases the sensitivity of the tumor cells to the SERCA inhibition induced by the SERCA inhibitor. Objective solid tumor response can be measured or monitored e.g., using the Response Evaluation Criteria in Solid Tumors (RECIST); cf. Therrasse et al. 2000, and Eisenhauer et al. 2009. The dose regimen of said at least one SERCA inhibitor and/or of said at least one Calmodulin antagonist (dose amount at each administration and/or frequency of administration of each dose) should also be adjusted so as to limit the induced toxicity to a level that is acceptable for the health of the subject(s).

The SERCA inhibitor in combination with a Calmodulin antagonist for use according to the present invention may be also combination wherein the SERCA inhibitor and the Calmodulin antagonist are formulated together in the same pharmaceutical composition.

In an embodiment, the SERCA inhibitor and the Calmodulin antagonist in combination for use according to the present invention are administered orally, intravenously, intramuscularly.

Preferred combinations of active ingredients according to the invention are those comprising thapsigargin or a prodrug thereof such as 8-O-(12Aminododecanoyl)-8-O debutanoylthapsigargin (G202) and the peptide prodrug L12ADT as SERCA inhibitor and trifluoperazine or Ophobolin A as Calmodulin antagonist.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

All publications and patents cited in this disclosure are incorporated by reference in their entirety. To the extent the material incorporated by reference contradicts or is inconsistent with this specification, the specification will supersede any such material.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having". When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

As described herein, "preferred embodiment" means "preferred embodiment of the present invention". Likewise, as described herein, "various embodiments" and "another embodiment" means "various embodiments of the present invention" and "another embodiment of the present invention", respectively.

### EXAMPLEs

### Example 1: The influence of Sec62 protein content in lung cancer:

The influence of Sec62 protein content in lung cancer on survival of patients was analyzed by the Kaplan-Meier method and a log-rank-test. To elucidate the underlying pathophysiological functions of Sec62, calcium imaging techniques, real time cell analysis and cell migration assays were used. Furthermore, the effects of treatment with Calmodulin antagonists triflouoperazine (TFP) and ophiobolin A on cellular calcium homeostasis, cell growth and cell migration were compared to siRNA mediated Sec62 depletion or expression of a mutated *SEC62* variant in human cell culture. BlAcore analysis was employed to study the calcium-sensitive interaction of Sec62 with the Sec61 complex.

### Results

Sec62 protein overproduction has a significant negative effect on patients' survival in lung cancer. Therefore, Sec62 protein has been postulated to be an interesting therapeutic target. On the molecular level, a regulatory function of Sec62 protein on the major calcium leak channel of the endoplasmic reticulum, the Sec61-channel, was postulated by the observation of a direct and calcium sensitive interaction. A calcium binding motive in Sec62 protein is shown to be essential for its molecular function. It has been found by the present inventors that treatment of cells with Calmodulin antagonists mimicked Sec62 depletion phenotypes, inhibited cell migration and rendered the cells sensitive for thapsigargin (SERCA inhibitor) treatment.

### Conclusions

Hence the present inventors have found that Sec62 protein function as a modulator of Ca²⁺Calmodulin binding to the major ER calcium leak channel. Furthermore the present inventors have found and shown that Calmodulin antagonists have a similar effect on tumor cells compared to *SEC62* gene silencing. Thus targeting Sec62 overproducing tumors using Calmodulin antagonists in combination with SERCA inhibitors (e.g. thapsigargin) is novel personalized therapeutic strategy for the treatment of tumors that overexpress *SEC62* gene and overproduce Sec62 protein.

### Example 2:Sec62 protein level in cancer tissue predicts survival rate of NSCLC patients

Detection of *SEC62* amplification and overexpression in non-small cell lung cancer (NSCLC) has been disclosed in reference [5]. The present inventors have tested whether a lower Sec62 protein content in cancer tissue was associated with longer patient survival, i.e., if Sec62 can indeed serve as a prognostic marker.

Using the Kaplan-Meier method and log-rank test, the inventors investigated the association between the rSec62 values of 70 NSCLC patients that were mentioned in the previous publication [5] with these patients' survival starting from the date of diagnosis.

Patients were divided into two groups based on their rSec62 value, applying thresholds of 2.1 (all patients, Figure 1A), 3 (squamous cell carcinoma (SCC) patients, Figure 1B), and 1.85 (adeno cell carcinoma (AC) patients, Figure 1C) in each case representing the mean rSec62 value of the respective group.

Survival analyses were visualized using Kaplan-Meier diagrams. Using the log-rank-test, the inventors observed a highly significant survival benefit in the low rSec62 group compared to the high rSec62 group, among all lung cancer patients as well as for SCC patients separately; however the survival of AC patients showed only a tendency but no significant dependency on rSec62 values (*P* = 0.001 for all NSCLC patients, *P* = 0.001 for SCC patients, *P* = 0.054 for AC patients).

Subsumed these data indicate the high clinical relevance of the Sec62 protein level majorly for SCC of the lung which is even more important in the context of the fact that the increased Sec62 protein level also protects tumor cells from the potential therapeutic use of thapsigargin[12].

### Example 3: Treatment with Calmodulin antagonists mimicks changes in the cytosolic calcium concentration as induced by SEC62 silencing

*SEC62* silencing is one possibility to overcome the protective effect of *SEC62* overexpression against thapsigargin, as *SEC62* silencing leads to an increase in cytosolic calcium concentration, as well as enhanced calcium leakage from the ER in combination with thapsigargin, as was shown by live cell calcium imaging[12]. The present inventors have also discovered a crucial influence of Calmodulin on ER calcium homeostasis, namely limiting ER calcium leakage by calcium-dependent binding to the Sec61-complex [16, 17]. As the therapeutic use of siRNAs still suffers from the problem of delivery, the present inventors considered whether the Sec62 protein regulates Calmodulin binding to the Sec61-complex, or if it may itself modulate the Sec61 complex as this would allow the therapeutic use of Calmodulin antagonists to counter the increased Sec62 protein level. To do this, the inventors have examined the effects on calcium homeostasis following treatment with the Calmodulin antagonists trifluoperazine (TFP) and ophiobolin A in comparison to the effects of siRNA mediated Sec62 depletion.

All the three treatments resulted in a comparable increase of cytosolic calcium in the absence of and in combination with thapsigargin treatment. These results strongly prove that that a similar molecular mechanism leads to deregulation of cellular calcium homeostasis after *SEC62* silencing and after treatment with Calmodulin antagonists.

Therefore, the inventors next considered whether the effect of Sec62 on ER Ca²⁺ leakage could be linked to the Ca²⁺ permeable Sec61 complex as it was shown before for the effects of TFP or ophiobolin A[16, 18]. To address this question, the inventors treated HeLa cells for 96 h with *SEC62* siRNA, *SEC61A1* siRNA, *SEC62* plus *SEC61A1* siRNA, or a negative-control siRNA. The results of live cell Ca²⁺ imaging experiments for cells with *SEC61A1* or *SEC62* siRNA confirmed the previous observations (Figure 2B) [12, 18]. Simultaneous silencing of *SEC61A1* and *SEC62* by siRNA, however, had an inhibitory effect on the *SEC62* silencing-induced Ca²⁺ efflux (Figure 2B). According to western blot analysis, the silencing efficiency of both siRNAs was above 80% (Figure 2C). Thus, the present inventors have established that Sec62 protein contributes to reducing Ca²⁺ leakage from the ER at the level of the Sec61 complex, presumably by direct interaction.

To directly demonstrate the putative interaction of Sec62 with Sec61a and to identify the Sec62 binding site, the present inventors have carreid out peptide binding experiments. Peptide spots that correspond to human Sec61a1 were synthesized on cellulose membranes. The peptides consisted of 12 amino acid residues with an overlap of 10 residues with adjacent peptides and were incubated with the C-terminal cytosolic domain of the double-spanning membrane protein Sec62.

The C-terminal domain of Sec62 (Sec62C) preferentially bound to the N-terminal peptide of Sec61a (amino acid residues 1-16) (Figure 2D, insert). In subsequent surface plasmon resonance spectroscopic (SPR) analysis (Figure 2D), the interaction of Sec62C with the N-terminal peptide of Sec61a was confirmed. Next, the inventors have analyzed if there is a Ca²⁺ effect for the observed interaction of Sec62C with Sec61a (Figure 2D). Sec62C showed more pronounced binding to Sec61a in the absence of Ca²⁺ than in its presence. *In silico* analysis of the Sec62 sequence identified a potential EF hand in C-terminal domains of vertebrate Sec62 (Figure 3A) that may explain this Ca²⁺ effect (amino acid residues 308-319).

### Example 4: A mutation in a predicted calcium-binding motif leads to a dominant-negative phenotype in cell migration and ER calcium leakage

The inventors previously showed that Sec62 depletion inhibits the spread of metastatic tumor cells and raises the cells' sensitivity to calcium-driven ER stress. By introducing the mutation D308A in the predicted calcium-binding motif within the C-terminus of Sec62, the inventors confirmed the function of Sec62 in regulating ER calcium homeostasis (Figure 3A). The expression of the plasmid-encoded *SEC62-*WT gene or *SEC62*_{D308A} gene was confirmed by quantitative western blot analysis of the stably transfected HEK293 cell lines. The inventors observed a nearly 5-fold increased Sec62 protein content in the pSEC62-WT and a more than 2-fold increased Sec62 protein content in the p*SEC62_{D308A}*-carrying cell line compared to the control plasmid-transfected cell line (Figure 3B). The inventors compared stably transfected HEK293 cells overexpressing a plasmid-encoded mutant *SEC62 gene* (p*SEC62_{D308A}*-IRES-GFP) or a *SEC62* wild-type gene (p*SEC62-*IRES-GFP) with respect to cell migration (Figure 3C), sensitivity to thapsigargin treatment (Figure 3D), and thapsigargin-induced calcium leakage from the ER (Figure 3E). Overproduction of wild-type Sec62 led to increased migration potential, as observed previously.⁵ In contrast, overproduction of mutant Sec62 protein reduced cell migration in a manner similar to *SEC62* silencing (Figure 3C). The same dominant-negative effect of *SEC62*_{D308A} expression was also observed regarding the cells' sensitivity to thapsigargin treatment (Figure 3D) and the thapsigargin-induced calcium leakage from the ER (Figure 3E). Overall, overexpression of WT *SEC62* did not affect cell growth or ER calcium leakage, whereas *SEC62*_{D308A} overexpression led to a phenotype comparable to that previously shown for *SEC62* silencing. These experiments clearly point to a direct influence of the predicted EF-hand motif of Sec62 on ER calcium homeostasis, and its direct connection to the observed phenotypes.

### Example 5: HeLa and HEK293 cells are more sensitive to trifluoperazine (TFP) treatment than PC3 cells

To study the influence of TFP and ophiobolin A on cellular processes other than calcium homeostasis, the inventors first analyzed the proliferation of PC3 (non-overproducing prostate cancer cell line) and HeLa cells in the presence of these two Calmodulin antagonists. The inventors also analyzed HEK293 cells (non overproducing cell line) with respect to their TFP sensitivity. PC3 and HeLa cells showed the same sensitivity to ophiobolin A; both cell lines showed normal growth behavior up to a concentration of 500 nM, whereas higher concentrations significantly inhibited cell growth (Figure 4A). In contrast, PC3 cells tolerated TFP up to concentrations of 24 µM, whereas HeLa-cells showed time-limited growth inhibition between 24-60 h after addition of the compound, indicating that HeLa cells were more sensitive to TFP treatment than PC3 cells (Figure 4B). HEK293 cells showed normal proliferation with up to 8 µM TFP in the medium, while cell growth was almost completely prevented with higher concentrations. Based on these findings, we used concentrations of up to 250 nM ophiobolin A and up to 8 µM of TFP as non-growth-inhibiting conditions for all cell lines in the following experiments. Interestingly, the HeLa and HEK293 cells, which were more sensitive to TFP treatment, also showed lower Sec62 protein content compared to PC3 cells. This was not due to a lower ER content of these cells, since the analyzed cell lines showed similar protein content of the ER chaperone BiP (Figure 4C). Together with the previous finding that Sec62 protein level is crucial for cell tolerance against thapsigargin-induced ER stress[12], the present findings further affirmed the direct role of Sec62 in the cellular response to calcium-driven ER stress.

### Example 6: Treatment with Calmodulin antagonists and SEC62 silencing result in comparable cellular phenotypes

Next, the inventors wanted to know if a strongly reduced migration potential and increased sensitivity to thapsigargin-induced ER stress could also be caused by TFP or ophiobolin A treatment. First, the inventors studied cell migration of PC3 and HeLa cells in the presence of increasing amounts of ophiobolin A or TFP. The inventors found dose-dependently reduced cell migration for both cell lines with both substances (Figure 5A). Again, HeLa cells were more sensitive to the treatments compared to PC3 cells. To confirm the results, the inventors also tested different human lung (H1299, A549, and BC01) and thyroid cancer cell lines (BHT101 and ML1) The inventors previously reported reduced migration in these cell lines after transfection with *SEC62* siRNA; here inventors found that 4 µM TFP or 100 nM ophiobolin A had the same effect in each tested lung and thyroid cancer cell line, namely a strong inhibition of cell migration without affecting cell proliferation.

Since Sec62 depletion by siRNA transfection alone was sufficient to block cell migration in the previous experiments [11], the inventors next asked if *SEC62* overexpression would rescue the migration defect of ophiobolin A or TFP-treated cells. HEK293 cells were used, which did not migrate in the assay without treatment but could be stimulated to migration by addition of 12-O-tetradecanoylphorbol 13-acetate (TPA), a drug that down-regulates agonist-driven calcium release from the ER [19] and stimulates cell migration [20, 21]. The inventors compared HEK293 cells stably transfected with a pIRES-*GFP* vector (control plasmid) with HEK293 cells stably overexpressing a plasmid-encoded *SEC62 gene* (p*SEC62*-IRES-GPF). Migration of un-transfected and control-transfected HEK293 cells were completely inhibited by 100 nM ophiobolin A or 8 µM TFP (Figure 5A). However, cells overexpressing *SEC62* still showed migration under these conditions (Figure 5B and C), indicating that the Sec62 protein content caused higher cell resistance against treatment with Calmodulin antagonists. Quantitative western blot analysis confirmed the approximately four-fold increase in Sec62 protein content in the p*SEC62*-WT-carrying HEK293 cells (Figure 5D). These observations supported the suggestion that the influence of Sec62 on cell migration is calcium dependent.

### Example 7: Growth inhibition induced by Calmodulin antagonists is strengthened by Sec62 depletion

Next the inventors wanted to investigate whether the observations regarding cell migration were also true for the second phenotype-increased cell sensitivity to thapsigargin treatment after *SEC62* silencing. The inventors first transfected PC3 cells with control siRNA or siRNA specifically directed against the *SEC62* mRNA, followed by treatment with 10 nM thapsigargin either in the presence of 8 µM TFP or 0.1% DMSO (solvent control) and observed cell growth. Again, this experiment showed higher sensitivity to thapsigargin treatment in Sec62-depleted cells, and a similar behavior of control siRNA-transfected cells after TFP treatment, indicated by a slightly weaker decline in the growth rate (Figure 6A and B). Combined treatment with *SEC62* siRNA and 8µM TFP resulted in even stronger growth inhibition, indicating an additive effect of *SEC62* silencing and Calmodulin antagonist treatment (Figure 6A and B). This additive effect also appeared with respect to cell migration (Figure 6C and D). Taken together, these results indicate that growth inhibition by the treatment with Calmodulin antagonists and by the reduction of cellular Sec62 protein level occur by the same mechanisms, providing valuable hints towards the function of Sec62 under cellular stress conditions.

The examples were performed using the following cell culture and tissue samples

Cell lines PC3 (DSMZ no. ACC 465), HeLa (DSMZ no. ACC 57) and HEK293 (DSMZ no. ACC 305) were cultured at 37°C in DMEM medium (Gibco Invitrogene, Karlsruhe, Germany) containing 10% FBS (Biochrom, Berlin, Germany) and 1% penicillin/streptomycin (PAA, Pasching, Austria), in a humidified environment with 5% CO₂. We used stably transfected HEK293 cell lines expressing plasmid-encoded wild-type *SEC62 gene* (p*SEC62*-IRES-GPF) or an empty control plasmid (pIRES-GPF) as already published.[5] A plasmid encoding a D308A point mutation of *SEC62* (p*sec62D308A*-IRES-GPF) was generated using the QuikChange Site-Directed Mutagenesis Kit (Stratagene) following the manufacturer's instructions. The plasmid was sequenced to confirm the point mutation. Also for this plasmid a stably transfected cell line was generated by transfecting 2.4 x 10⁵ HEK293 cells in a 6-well plate using FuGeneHD Reagent (Qiagen) following the manufacturer's instruction. After 72 hours, medium was changed to normal culture medium containing 1% G418 and cells were further cultured until a selection was reached. Then cells were harvested, diluted to a density of 1 cell per 100 µl and 100 µl per well was seeded to a 96-well plate in medium containing 1% G418. Clones originated from a single cell were selected and analyzed with respect to Sec62 protein content. Further cultivation and all experiments using stably transfected cell lines were performed in normal growth medium containing 1% G418.

The Sec62 concentration in cancerous and tumor-free lung tissue of 70 NSCLC patients with pathologically confirmed AC or SCC were measured by western blot using β-actin as a loading control in the previous study and calculated a relative Sec62 (rSec62=[Sec62ₜᵤₘₒᵣ/b-actinₜᵤₘₒᵣ]/ [Sec62_{tumorfree}/b-actin_{tumor-free}]) protein content elevation in the tumor.[5] Either all patients (n=70) or the subgroups of AC (n=35) or SCC (n=35) patients were divided into two groups based on the mean rSec62 value of the respective group, and survival analyses were performed using the Kaplan-Meier method and log-rank-test.

### Protein quantification in cells by western blot

Proteins were quantified from the lysate of 2 × 10⁵ cultured cells by western blot analysis. An affinity-purified polyclonal rabbit anti-peptide antibody directed against the COOH terminus of human Sec62, a polyclonal rabbit anti-BiP antibody, a polyclonal rabbit anti-peptide antibody directed against the C-terminus of human Sec61a, and a polyclonal anti-β-actin antibody (Cell Signaling Biotechnology, FL-335) were used. The primary antibodies were visualized with an ECL^{™} Plex goat anti-rabbit IgG-Cy5 or ECL^{™} Plex goat anti-mouse IgG-Cy3 conjugate (GE Healthcare, Munich, Germany) and the Typhoon-Trio imaging system (GE Healthcare) in combination with the Image Quant TL software 7.0 (GE Healthcare). We determined the ratio of Sec62, Sec61a, or BiP to β-actin.

### Silencing of gene expression by siRNA

For gene silencing, 5.4 × 10⁵ cells were seeded in 6-cm dishes, in normal culture medium. The cells were transfected with *SEC62*-UTR siRNA (CGUAAAGUGUAUUCUGUACtt; Ambion, TX, USA), *SEC62* siRNA (GGCUGUGGCCAAGUAUCUUtt; Ambion), *SEC61A1* siRNA (GGAAUUUGCCUGCUAAUCAtt, Qiagen, Hilden, Germany), or control siRNA (AllStars Neg. Control siRNA; Qiagen) using HiPerFect Reagent (Qiagen) following the manufacturer's instructions. After 24 h, the medium was changed and the cells were transfected a second time. Silencing efficiency was evaluated by western blot analysis. The maximum silencing effect was seen 72 h (*SEC62* siRNAs) or 96 h (*SEC61A1* siRNA) after the first transfection.

### Real-time cell proliferation analysis

The xCELLigence SP system (Roche Diagnostics GmbH, Mannheim, Germany) was used for the real-time analysis of cell proliferation. In this system, 1.0 × 10⁴ or 2.0 × 10⁴ either stably transfected HEK293 cell lines as described above or untreated HEK293-, PC3- or HeLa-cells or PC3-cells pretreated with siRNA in 6-cm dishes as described above were seeded into a 96-well e-plate (Roche Diagnostics GmbH) according to the manufacturer's instructions. The seeding of siRNA pretreated cells occurred 24 hr after the second transfection. If cells were treated with thapsigargin, TFP or ophiobolin A, this occurred at least 4 hr after seeding onto the plates. Cell proliferation was monitored for 53-96 h, and data evaluated with RTCA 1.2 software (Roche Diagnostics GmbH).

### Peptide spot binding assay

13 Peptides scanning the N-terminus of human Sec61a were synthesized on cellulose membranes via carboxy-terminal attachment as described.[16, 45] The peptides consisted of 12 amino-acid residues with an overlap of 10 residues with adjacent peptides and were incubated with Sec62-C-6His (1 µM) purified from *E. coli* as described previously[10] in binding buffer (30mM Tris-HCl pH 7.4, 170 mM NaCl, 6.4 mM KCl, 5% sucrose, 0.05% Tween20). After washing the membranes two times with binding buffer bound protein was detected using anti-His-POD coupled antibody (1:1000, Qiagen), again washed two times with binding buffer, followed by incubation with ECL (GE Healthcare) and subsequently visualized with a lumi-imaging system (Roche Diagnostics GmbH).

### Surface plasmon resonance spectroscopy

Surface plasmon resonance spectroscopy was performed in a BIAlite upgrade system (BIACORE). Peptides representing the N-terminus of Sec61 (AIKFLEVIKPFC) in the measuring cell or the N-terminus of TRAM (VLSHEFELQNGADC) in the control cell were immobilized on a CM5 sensor chip using ligand-thiol-coupling according to the manufacturer's protocol. Measurements were performed at a flow rate of 10 µl/min in 10 mM HEPES-KOH, pH 7.4, 150 mM NaCI, 2 mM MgCI, 6.4 mM KCl and 0.005% surfactant (buffer minus Ca²⁺). For interaction analysis Sec62-C-6His (1 µM) purified from *E*. *coli* as described previously[10] in the buffer minus Ca²⁺ or in the same buffer containing 2 mM Ca²⁺ or the calcium containing buffer alone was passed over the chip. Response units are shown as a difference between measuring- and control cell. The analysis was carried out by employing BIA evaluation software version 3.1 (BIACORE) using 1:1 binding models and mass transfer.

### Migration potential analysis

Migration was tested with the BD Falcon FluoroBlok system (BD, NJ, USA) in 24-well inserts. A total of 2.5 × 10⁴ either stably transfected HEK293 cell lines as described above or untreated PC3- or HeLa-cells were loaded into this system in normal medium containing 0.5% FBS. If DMSO, TFP or ophiobolin A was used these drugs were added to the top and to the bottom chamber in the concentrations given in the figure. The inserts were placed in medium with 10% FBS as a chemo-attractant. After 72 h, the cells were fixed with methanol and stained with DAPI, and migrating cells were analyzed on the backside of the membrane by fluorescence microscopy.

### Live-cell calcium imaging

For live-cell calcium imaging, HeLa cells were loaded with 4 mM FURA-2 AM (Molecular Probes, Eugene, OR, USA) in DMEM for 45 min at room temperature as previously described.[46, 47] Two washes were performed with a calcium-free buffer (140 mM NaCI, 5 mM KCl, 1 mM MgCl2, 0.5 mM EGTA, and 10 mM glucose in 10 mM HEPES-KOH at pH 7.35) and the experiments were carried out in the same solution. A ratiometric measurement was performed for 3 min to determine the initial cytosolic [Ca²⁺], then thapsigargin (1 µM) was added and the measurement was continued. Cells differently pretreated as described in the text were compared with respect to initial cytosolic [Ca²⁺] and thapsigargin-induced changes in cytosolic [Ca²⁺]. Data were collected on an iMIC microscope and the polychromator V (Till Photonics, Graefelfing, Germany) by alternately exciting at 340 and 380 nm, and measuring the emitted fluorescence at 510 nm (dichroic, DCLP410; emitter filter LP470; Till Photonics). Images containing 50-60 cells/frame were sampled every 3 sec. FURA-2 signals were recorded as a F340/F380 ratio, where F340 and F380 correspond to the background-subtracted fluorescence intensities at 340 and 380 nm, respectively. Cytosolic [Ca²⁺] was estimated from ratio measurements using an established calibration method.[48] Data were analyzed using Excel 2007 and Origin 6.1

### References

1. Ferlay J, Shin HR, Bray F, Forman D, Mathers C, Parkin DM: Estimates of worldwide burden of cancer in 2008: GLOBOCAN 2008. Int J Cancer 2010, 127:2893-2917.
2. Beaglehole R, Bonita R: Global public health: a scorecard. Lancet 2008, 372:1988-1996.
3. Bray FI, Weiderpass E: Lung cancer mortality trends in 36 European countries: secular trends and birth cohort patterns by sex and region 1970-2007. Int J Cancer 2009, 126:1454-1466.
4. Herbst RS, Heymach JV, Lippman SM: Lung cancer. N Engl J Med 2008, 359:1367-1380.
5. Linxweiler M, Linxweiler J, Barth M, Benedix J, Jung V, Kim YJ, Bohle RM, Zimmermann R, Greiner M: Sec62 bridges the gap from 3q amplification to molecular cell biology in non-small cell lung cancer. Am J Pathol 2012, 180:473-483.
6. Panzner S, Dreier L, Hartmann E, Kostka S, Rapoport TA: Posttranslational protein transport in yeast reconstituted with a purified complex of Sec proteins and Kar2p. Cell 1995, 81:561-570.
7. Meyer H-A, Grau H, Kraft R, Kostka S, Prehn S, Kalies K-U, Hartmann E: Mammalian Sec61 is associated with Sec62 and Sec63. J Biol Chem 2000, 275:14550-14557.
8. Lakkaraju AK, Thankappan R, Mary C, Garrison JL, Taunton J, Strub K: Efficient secretion of small proteins in mammalian cells relies on Sec62-dependent posttranslational translocation. Mol Biol Cell 2012, 23:2712-2722.
9. Lang S, Benedix J, Fedeles SV, Schorr S, Schirra C, Schauble N, Jalal C, Greiner M, Hassdenteufel S, Tatzelt J, et al: Different effects of Sec61alpha, Sec62 and Sec63 depletion on transport of polypeptides into the endoplasmic reticulum of mammalian cells. J Cell Sci 2012, 125:1958-1969.
10. Muller L, Diaz de Escauriaza M, Lajoie P, Theis M, Jung M, Muller A, Burgard C, Greiner M, Snapp EL, Dudek J, Zimmermann R: Evolutionary Gain of Function for the ER Membrane Protein Sec62 from Yeast to Humans. Mol Biol Cell 2010, 21:691-703.
11. Greiner M, Kreutzer B, Jung V, Grobholz R, Hasenfus A, Stöhr RF, Tornillo L, Dudek J, Stöckle M, Unteregger G, et al: Silencing of the SEC62 gene inhibits migratory and invasive potential of various tumor cells. Int J Cancer 2011, 128:2284-2295.
12. Greiner M, Kreutzer B, Lang S, Jung V, Adolpho C, Unteregger G, Zimmermann R, Wullich B: Sec62 protein content is crucial for the ER stress tolerance of prostate cancer. The Prostate 2011, 71:1074-1083.
13. Calfon M, Zeng H, Urano F, Till JH, Hubbard SR, Harding HP, Clark SG, Ron D: IRE1 couples endoplasmic reticulum load to secretory capacity by processing the XBP-1 mRNA. Nature 2002, 415:92-96.
14. Nishitoh H, Matsuzawa A, Tobiume K, Saegusa K, Takeda K, Inoue K, Hori S, Kakizuka A, lchijo H: ASK1 is essential for endoplasmic reticulum stress-induced neuronal cell death triggered by expanded polyglutamine repeats. Genes Dev 2002, 16:1345-1355.
15. Huang JB, Kindzelskii AL, Clark AJ, Petty HR: Identification of channels promoting calcium spikes and waves in HT1080 tumor cells: their apparent roles in cell motility and invasion. Cancer Res 2004, 64:2482-2489.
16. Erdmann F, Schauble N, Lang S, Jung M, Honigmann A, Ahmad M, Dudek J, Benedix J, Harsman A, Kopp A, et al: Interaction of calmodulin with Sec61alpha limits Ca2+ leakage from the endoplasmic reticulum. Embo J 2011, 30:17-31.
17. Harsman A, Kopp A, Wagner R, Zimmermann R, Jung M: Calmodulin regulation of the calcium-leak channel Sec61 is unique to vertebrates. Channels (Austin) 2011, 5:293-298.
18. Lang S, Schauble N, Cavalie A, Zimmermann R: Live cell calcium imaging combined with siRNA mediated gene silencing identifies Ca(2)(+) leak channels in the ER membrane and their regulatory mechanisms. J Vis Exp 2011:e2730.
19. Chen L, Meng Q, Jing X, Xu P, Luo D: A role for protein kinase C in the regulation of membrane fluidity and Ca2+ flux at the endoplasmic reticulum and plasma membranes of HEK293 and Jurkat cells. Cellular Signalilng 2011, 23:497-505.
20. Nabeshima K, Komada N, Kishi J, Koita H, Inoue T, Hayakawa T, Koono M: TPA-enhanced invasion of Matrigel associated with augmentation of cell motility but not metalloproteinase activity in a highly metastatic variant (L-10) of human rectal adenocarcinoma cell line RCM-1. Int J Cancer 1993, 55:974-981.
21. Lin CW, Shen SC, Chien CC, Yang LY, Shia LT, Chen YC: 12-O-tetradecanoylphorbol-13-acetate-induced invasion/migration of glioblastoma cells through activating PKCalpha/ERK/NF-kappaB-dependent MMP-9 expression. J Cell Physiol 2010, 225:472-481.
22. Lee J, Ishihara A, Oxford G, Johnson B, Jacobson K: Regulation of cell movement is mediated by stretch-activated calcium channels. Nature 1999, 400:382-386.
23. Ridley AJ, Schwartz MA, Burridge K, Firtel RA, Ginsberg MH, Borisy G, Parsons JT, Horwitz AR: Cell migration: integrating signals from front to back. Science 2003, 302:1704-1709.
24. Sjaastad MD, Nelson WJ: Integrin-mediated calcium signaling and regulation of cell adhesion by intracellular calcium. Bioessays 1997, 19:47-55.
25. Schauble N, Lang S, Jung M, Cappel S, Schorr S, Ulucan O, Linxweiler J, Dudek J, Blum R, Helms V, et al: BiP-mediated closing of the Sec61 channel limits Ca(2+) leakage from the ER. Embo J 2012, 31:3282-3296.
26. Wittke S, Dunnwald M, Johnsson N: Sec62p, a component of the endoplasmic reticulum protein translocation machinery, contains multiple binding sites for the Sec-complex. Mol Biol Cell 2000, 11:3859-3871.
27. Christie RJ, Nishiyama N, Kataoka K: Delivering the code: polyplex carriers for deoxyribonucleic acid and ribonucleic acid interference therapies. Endocrinology 2009, 151:466-473.
28. Jackson AL, Linsley PS: Recognizing and avoiding siRNA off-target effects for target identification and therapeutic application. Nat Rev Drug Discov 2010, 9:57-67.
29. Koehn S, Schaefer HW, Ludwig M, Haag N, Schubert US, Seyfarth L, Imhof D, Markert UR, Poehlmann TG: Cell-specific RNA interference by peptide-inhibited-peptidase-activated siRNAs. J RNAi Gene Silencing 2010, 6:422-430.
30. Schmidt C: RNAi momentum fizzles as pharma shifts priorities. Nat Biotechnol 2011, 29:93-94.
31. Bonetta L: RNA-based therapeutics: ready for delivery? Cell 2009, 136:581-584.
32. Coticchia CM, Revankar CM, Deb TB, Dickson RB, Johnson MD: Calmodulin modulates Akt activity in human breast cancer cell lines. Breast Cancer Res Treat 2009, 115:545-560.
33. Hwang YP, Jeong HG: Metformin blocks migration and invasion of tumour cells by inhibition of matrix metalloproteinase-9 activation through a calcium and protein kinase Calpha-dependent pathway: phorbol-12-myristate-13-acetate-induced/extracellular signal-regulated kinase/activator protein-1. Br J Pharmacol 2010, 160:1195-1211.
34. Jung HJ, Kim JH, Shim JS, Kwon HJ: A novel Ca2+/calmodulin antagonist HBC inhibits angiogenesis and down-regulates hypoxia-inducible factor. J Biol Chem 2010, 285:25867-25874.
35. Yuan K, Jing G, Chen J, Liu H, Zhang K, Li Y, Wu H, McDonald JM, Chen Y: Calmodulin mediates Fas-induced FADD-independent survival signaling in pancreatic cancer cells via activation of Src-extracellular signal-regulated kinase (ERK). J Biol Chem 2011, 286:24776-24784.
36. Polischouk AG, Holgersson A, Zong D, Stenerlow B, Karlsson HL, Moller L, Viktorsson K, Lewensohn R: The antipsychotic drug trifluoperazine inhibits DNA repair and sensitizes non small cell lung carcinoma cells to DNA double-strand break induced cell death. Mol Cancer Ther 2007, 6:2303-2309.
37. Satyamoorthy K, Perchellet JP: Modulation by adriamycin, daunomycin, verapamil, and trifluoperazine of the biochemical processes linked to mouse skin tumor promotion by 12-O-tetradecanoylphorbol-13-acetate. Cancer Res 1989, 49:5364-5370.
38. Carpenter WT, Jr., Davis JM: Another view of the history of antipsychotic drug discovery and development. Mol Psychiatry 2012.
39. Shen WW: A history of antipsychotic drug development. Compr Psychiatry 1999, 40:407-414.
40. Ghantous A, Gali-Muhtasib H, Vuorela H, Saliba NA, Darwiche N: What made sesquiterpene lactones reach cancer clinical trials? Drug Discov Today 2010, 15:668-678.
41. Christensen SB, Skytte, D.M., Denmeade, S.R., Dionne, C., Møller, J.V., Nissen, P., Isaacs, J.T.: A Trojan Horse in Drug Development: Targeting of Thapsigargins Towards Prostate Cancer Cells. Anticancer Agents Med Chem 2009, 9:276-294.
42. Huang J-K, Huang C-C, Lu T, Chang H-T, Lin K-L, Tsai J-Y, Liao W-C, Chien J-M, Jan C-R: Effect of MK-886 on Ca2+ Level and Viability in PC3 Human Prostate Cancer Cells. Basic Clin Pharmacol Toxicol 2009, 104:441-447.
43. Denmeade SR, Isaacs JT: The SERCA pump as a therapeutic target: making a "smart bomb" for prostate cancer. Cancer Biol Ther 2005, 4:14-22.
44. Denmeade SR, Mhaka AM, Rosen DM, Brennen WN, Dalrymple S, Dach I, Olesen C, Gurel B, Demarzo AM, Wilding G, et al: Engineering a prostate-specific membrane antigen-activated tumor endothelial cell prodrug for cancer therapy. Sci Transl Med 2012, 4:140ra186.
45. Hilpert K, Winkler DF, Hancock RE: Peptide arrays on cellulose support: SPOT synthesis, a time and cost efficient method for synthesis of large numbers of peptides in a parallel and addressable fashion. Nat Protoc 2007, 2:1333-1349.
46. Aneiros E, Philipp S, Lis A, Freichel M, Cavalie A: Modulation of Ca2+ signaling by Na+/Ca2+ exchangers in mast cells. J Immunol 2005, 174:119-130.
47. Gross SA, Guzman GA, Wissenbach U, Philipp SE, Zhu MX, Bruns D, Cavalie A: TRPC5 is a Ca2+-activated channel functionally coupled to Ca2+-selective ion channels. J Biol Chem 2009, 284:34423-34432.
48. Lomax RB, Camello C, Van Coppenolle F, Petersen OH, Tepikin AV: Basal and physiological Ca(2+) leak from the endoplasmic reticulum of pancreatic acinar cells. Second messenger-activated channels and translocons. J Biol Chem 2002, 277:26479-26485.

## Claims

1. A combination of pharmaceutically active compounds comprising at least a SERCA inhibitor compound and at least a Calmodulin antagonist compound for use in the treatment of a tumor **characterized by** the overexpression of *SEC62* gene and/or by the overproduction of Sec62 protein

2. The combination for use according to claim 1 wherein the tumor is treated by a SERCA inhibitor and **characterized by** the overexpression of *SEC62* gene.

3. The combination for use according to claim 1 or 2 wherein the SERCA inhibitor is selected from sesquiterpene lactones and derivative thereof (compounds built from 3 isoprene units and containing a lactone ring) , in particular thapsigargin and prodrugs thereof such as 8-O-(12Aminododecanoyl)-8-O debutanoylthapsigargin (G202) and the peptide prodrug L12ADT and, artemisinin; hydroquinone based compounds such as 2,5-di(tert-butyl)hydroquinone (BHQ); 1,3-dibromo-2,4,6-tris (methyl-isothio-uronium) benzene.

4. The combination for use according to claim 1 or 2 or 3 wherein the Calmodulin antagonist is selected from trifluoperazine, Ophobolin A CGS 9343B (CAS 109826-27-9- Zaldaride maleate, W-13 isomer hydrochloride (CAS # 88519-57-7); W5 (CAS # 61714-25-8), E6 Berbamine (CAS # 73885-53-7), N-(6-Aminohexyl)-5-chloro-1-naphthalenesulfonamide Hydrochloride; Calmidazolium Chloride (CAS 57265-65-3); Fluphenazine-N-2-chloroethane·2HCl (CAS # 83016-35-7); CAS # 187937-24-2 (CAS # 187937-24-2); W-13 Isomer (CAS # 79127-24-5), Decyl Analog hydrochloride (CAS # 79127-24-5); Phenoxybenzamine (CAS # 63-92-3).

5. The combination for use according to any of the proceeding claims wherein the tumor is selected from non-small lung cell lung cancer, thyroid cancer, prostate cancer, head and neck cancer, multiple myeloma.

6. The combination for use according to any of the proceeding claims wherein the overproduction of Sec62 protein or the overexpression of *SEC62* gene in a tumor histological tissue of a patient is measured with respect to the tumor free histological tissue of the same patient.

7. The combination for use according to any of the proceeding claims wherein the overexpression of *SEC62* gene is measured by the analysis of *SEC62* mRNA content in a tumor cell and the analysis reveals a more than 1.5 fold increase in *SEC62* expression relative to tumor free histological tissue of the same patient and/or wherein the overproduction of Sec 62 protein is measured by the analysis of Sec62 protein level in a tumor cell and the analysis reveal a more than 1.5 fold increase in Sec62 protein level relative to tumor free tissue of the same patient.

8. The combination for use according to claim 7 wherein the *SEC62* mRNA is measured with RT-PCR or with in situ hybridization or with RNA array and the Sec62 protein is measured with Western Blot, histochemistry method or protein arrays.

9. The combination for use according to any of the proceeding claims wherein the SERCA inhibitor and the Calmodulin antagonist are administered simultaneously or sequentially or separately.

10. The combination for use according to any of the proceeding claims wherein the SERCA inhibitor and the Calmodulin antagonist are administered sequentially within an interval of 1 to 3 hours preferably, 1 hour.

11. The combination for use according to any of the proceeding claims wherein the SERCA inhibitor and the Calmodulin antagonist are formulated together in the same pharmaceutical composition.

12. The combination for use according to any of the proceeding claims wherein the SERCA inhibitor and the Calmodulin antagonist are administered orally, intravenously, intramuscularly.
